(19) Europäisches Patentamt
European Patent Office
Office européen des brevets

(11) Veröffentlichungsnummer: **0 163 607**
**B1**

(12) # EUROPÄISCHE PATENTSCHRIFT

(45) Veröffentlichungstag der Patentschrift: **08.11.89**

(51) Int. Cl.⁴: **C 07 C 103/38**, C 07 C 103/375, C 07 C 131/00, C 07 D 319/06, A 01 N 25/00

(21) Anmeldenummer: **85810241.1**

(22) Anmeldetag: **22.05.85**

(54) **Mittel zum Schützen von Kulturpflanzen vor der phytotoxischen Wirkung von herbizid wirksamen Chloracetaniliden.**

(30) Priorität: **28.05.84 CH 2612/84**
**22.11.84 CH 5590/84**
**25.01.85 CH 350/85**
**24.04.85 CH 1757/85**

(43) Veröffentlichungstag der Anmeldung:
**04.12.85 Patentblatt 85/49**

(45) Bekanntmachung des Hinweises auf die Patenterteilung:
**08.11.89 Patentblatt 89/45**

(84) Benannte Vertragsstaaten:
**AT BE CH DE FR GB IT LI NL**

(56) Entgegenhaltungen:
**EP-A- 0 046 666**
**EP-A- 0 140 271**
**BE-A- 535 830**
**BE-A- 569 109**
**GB-A- 2 031 420**
**GB-A- 2 078 521**
**US-A- 3 733 339**
**US-A- 3 950 335**
**US-A- 4 208 203**
**US-A- 4 269 618**

**CHEMICAL ABSTRACTS, Band 60, Nr. 4, 17. Februar 1964, Spalte 4136g - 4137c, Columbus, Ohio, US; S.L. DALAL et al.: "Potential local anaesthetics. III. Synthesis of basic N-[alpha-alkyl(or aryl) benzyl] acyl amides"**
**CHEMICAL ABSTRACTS, Band 58, Nr. 7, 1. April 1963, Spalte 6721c - 6722c, Columbus, Ohio, US; A.A.**

(73) Patentinhaber: **CIBA-GEIGY AG, Klybeckstrasse 141, CH-4002 Basel (CH)**

(72) Erfinder: **Martin, Henry, Dr., Schützenmattstrasse 52, CH-4051 Basel (CH)**

(56) Entgegenhaltungen: (Fortsetzung)
**AROYAN et al.: "Syntheses from chloromethyl derivatives of esters of o- and p-cresols"**
**CHEMICAL ABSTRACTS, Band 58, Nr. 3, 4. Februar 1963, Spalte 2402e-f, Columbus, Ohio, US; G.S. SIDHU et al.: "Synthesis and anticonvulsant activity of some N-phenethylacetamides"**
**CHEMICAL ABSTRACTS, Band 56, Nr. 9, 30. April 1962, Spalte 10006g, Columbus, Ohio, US; A.L. MNDZHOYAN et al.: "Amines and derivatives. XI. The preparation of some chloroacetamides and urethans"**
**JOURNAL CHEMICAL SOCIETY, 1962, Seiten 1420-1427; D.A.A. KIDD et al.: "The chemotherapy of amoebiasis. Some N-substituted dichloroacetamides"**
**CHEM. PHARM. BULL., Band 29, Nr. 1, 1981, Seiten 128-136; T. HAMADA et al.: "Photochemical synthesis of 1,2,3,4-tetrahydroisoquinolin-3-ones and oxindoles from N-chloroacetyl derivatives of benzylamines and anilines. Role of intramolecular exciplex formation and cis conformation of amide bonds"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 163 607 B1

**Beschreibung**

Die vorliegende Erfindung betrifft ein Mittel zum Schutz von Kulturpflanzen vor der phytotoxischen Wirkung von herbizid wirksamen Chloracetaniliden, welches als Herbizid-antagonisierenden Wirkstoff ein Acylamid-Derivat enthält, ferner ein Mittel welche neben diesem antagonisierenden Wirkstoff bereits das Herbizid enthalten und ein Verfahren zur selektiven Unkrautbekämpfung mittels dieser Herbizide und diesem Gegenmittel. Die Erfindung umfaßt auch Verfahren zur Herstellung der Acylamid-Derivate.

Es ist bekannt, daß Herbizide aus den verschiedensten Stoffklassen, wie Triazine, Harnstoffderivate, Carbamate, Thiolcarbamate, Halogenacetanilide, Halogenphenoxyessigsäuren usw. bei der Anwendung in wirksamer Dosis gelegentlich neben den zu bekämpfenden Unkräutern auch die Kulturpflanzen in gewissem Maße schädigen. Überdosen werden oft ungewollt und zufälligerweise appliziert, wenn sich Randzonen oder durch falsches Einschätzen der Breitenwirkung des Spritzgerätes. Es können klimatische Verhältnisse oder eine Bodenbeschaffenheit vorliegen, so daß die für normale Bedingungen empfohlene Herbizidmenge als Überdosis wirkt. Die Qualität des Saatgutes kann bei der Herbizidverträglichkeit auch eine Rolle spielen. Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, daß die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

Die direkte pre- oder postemergente Behandlung von Getreide und Mais mit Gegenmitteln als Antagonisten von Thiocarbamaten und anderen Herbizidklassen auf einer Anbaufläche ist in den Deutschen Offenlegungsschriften 2 141 586 und 2 218 097, sowie im US-Patent 3 867 444 beschrieben.

Ferner können Maispflanzen gemäß der deutschen Offenlegungsschrift 2 402 983 wirksam vor Schädigung durch Chloracetanilide geschützt werden, indem man dem Boden als Gegenmittel ein N-disubstituiertes Dichloracetamid zuführt. Derartige Verbindungen werden gemäß US-PS 4 137 070 auch als Antidotes bei herbiziden Thiocarbamaten verwendet oder gemäß DE-OS 2 828 265 und 2 828 293 als Antidotes gegen herbizide Acetanilide.

Es wurde nun gefunden, daß sich überraschenderweise eine Gruppe von Acylamid-Derivaten hervorragend dazu eignet, Kulturpflanzen gegen schädigende Wirkung von Agrarchemikalien, wie beispielsweise Pflanzenschutzmitteln, insbesondere Herbiziden, zu schützen. Diese Acylamid-Derivate werden daher im folgenden auch als «Gegenmittel», «Antidot» oder «Safener» bezeichnet.

Acylamid-Derivate, d.h. halogenacylierte Phenylalkylamine die zum Schützen von Kulturpflanzen gegen die schädigende Wirkung von herbizid wirksamen Chloracetaniliden geeignet sind entsprechen der Formel I

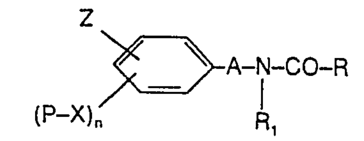
(I),

worin

X O oder S, SO, $SO_2$,

P Alkyl, Alkenyl, Alkinyl, Cycloalkyl, Alkoxyalkyl, Alkenoxyalkyl, Alkinoxyalkyl, Alkylthioalkyl, Alkenylthioalkyl, Alkinylthioalkyl, Alkylsulfinylalkyl, Alkylsulfonylalkyl, Halogenalkyl, Cyanalkyl, 2,2-Dialkoxy-alkyl, 1,3-Dioxolan-2-ylalkyl, 1,3-Dioxolan-4-ylalkyl, 2,2-Dialkyl-1,3-dioxolan-4-ylalkyl, 1,3-dioxan-yl-2-alkyl, 2-Benzpyranyl-alkyl, Alkoxycarbonyl oder Alkenyloxycarbonyl oder Tetrahydrofurfurylalkyl, die Gruppe

p–X auch Halogenalkyl

n 1–3

Z Wasserstoff, Alkyl, Halogen, Dioxymethylen, Alkoxy, Alkenyloxy, Alkinyloxy oder Cyanoalkyl

A einen geradkettigen, verzweigten oder cyclischen $C_1$–$C_8$-Kohlenwasserstoffrest, welcher unsubstituiert oder durch Alkoxy, Alkylthio, Fluor, Cyan oder Halogenalkyl substituiert sein kann

R Halogenalkyl oder Halogenalkenyl,

$R_1$ Wasserstoff, einen geradkettigen oder verzweigten, gesättigten oder ungesättigten $C_1$–$C_5$-Kohlenwasserstoffrest der unsubstituiert oder durch Alkoxy, poly-Alkoxy, Halogen, Cyan oder Trifluormethyl, substituiert sein kann, oder Alkylcycloalkyl, Dialkoxyalkyl, 1,3-Dioxolan-2-ylalkyl, 1,3-Dioxolan-4-ylalkyl, 1,3-Dioxan-2-ylalkyl, Furylalkyl, Tetrahydrofurylalkyl oder einen Rest $-NH-CCOR_2$ oder $-CH_2-COOR_2$ oder $-CH(CH_3)COOR_2$ oder einen Alkoxyiminoalkylrest$-CH(R_3)-C(R_4)=NOR_5$ wobei

$R_2$ Methyl, Äthyl, Propyl, Isopropyl oder Allyl,

$R_3$ und $R_4$ je Wasserstoff oder $C_1$–$C_4$ Alkyl und

$R_5$ Wasserstoff, $C_1$–$C_4$ Alkyl, $C_3$–$C_4$ Alkenyl oder $C_3$–$C_4$ Alkinyl bedeuten.

Unter Halogen selbst in den Definitionen sowie Halogen als Teil in Halogenalkoxy, Halogenalkyl oder Halogenalkenyl sind Fluor, Chlor, Brom und Jod, vorzugsweise jedoch Fluor, Chlor und Brom, insbesondere aber Chlor zu verstehen.

In den Definitionen ist unter Alkyl geradkettiges oder verzweigtes Alkyl zu verstehen; z.B.: Methyl, Äthyl, n-Propyl, i-Propyl oder die isomeren Butyl, Pentyl oder Hexyl.

Unter Alkoxy ist zu verstehen: Methoxy, Äthoxy, n-Propyloxy, i-Propyloxy oder die isomeren Butyloxy-, Pentyloxy- oder Hexyloxyreste, insbesondere aber Methoxy, Äthoxy oder i-Propyloxy.

Beispiele für ungesättigte Substituenten oder Substituententeile sind Vinyl, Vinyloxy, Allyl, Allyloxy, Propargyl, Propargyloxy, Methallyl, Methallyloxy, Butenyl, Butenyloxy, Butinyl, Butinyloxy, Chlorvinyl, Dichlorvinyl, Trichlorvinyl, 3,3,3-Trifluor-1-propenyl, 3,3,3-Trichlor-1-propinyl oder 2,3-Dichlorpropengyl.

Alkoxyalkylreste werden repräsentiert durch Methoxymethyl, Äthoxymethyl, Methoxyäthyl und Äthoxyäthyl, Propoxyäthyl, Isopropoxyäthyl, Butoxyäthyl, Allyloxyäthyl, insbesondere aber Methoxyäthyl. Halogenalkyl als Substituenten wie Halogenalkoxy oder Halogenalkylthio steht in der Regel für Chlormethyl, Fluormethyl, Difluormethyl, Trifluormethyl, Dichlormethyl, Trichlormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 2-Chlorpropyl, 1,1,2,3,3,3-Hexafluorpropyl, 3,3,3-Trichlorpropyl, 2,2,2-Trichloräthyl, 1-Chloräthyl, insbesondere aber Chlormethyl, Dichlormethyl, Trichlormethyl und 1-Chloräthyl.

Wegen ihrer Wirkung als Herbizidantagonisten sind diejenigen Acylamid-Derivate der Formel I aufgefallen, in denen

a) X Sauerstoff

b) P $C_1$–$C_6$-Alkyl; 2,2-Dialkoxyalkyl, 1,3-Dioxolan-2-ylalkyl,

c) n 1 oder 2

d) Z Wasserstoff

e) X–P Trifluormethyl

f) A ein Brückenglied $-CH_2-$, $-CH_2-CH_2-$, $-CH_2-CH_2-CH_2-$, 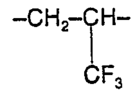

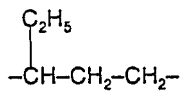 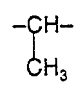 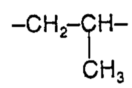 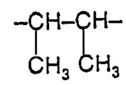 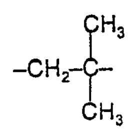 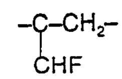

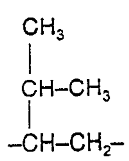 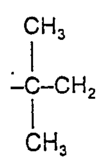 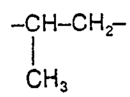 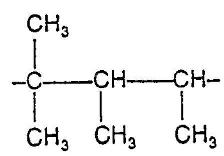 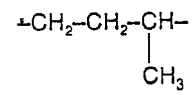

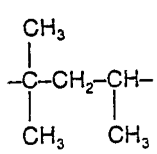 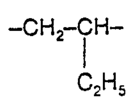 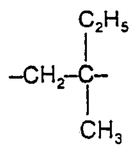 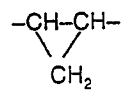 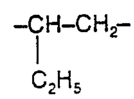

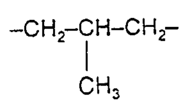 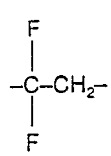 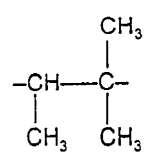 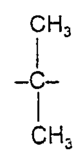 $-CH=CH-CH_2-$ 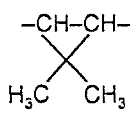

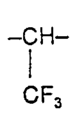 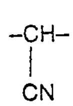 $-CHF-CH_2-$ 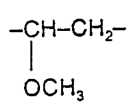

g) $R_1$ Wasserstoff, $C_1$–$C_6$-Alkyl, $C_2$–$C_4$-Alkenyl, $C_1$–$C_4$-Alkoxyalkyl, Tetrahydrofurfuryl oder $NH-COOC_2H_5$ oder $-CH_2-COOC_2H_5$

h) R $C_1$–$C_6$-Halogenalkyl bedeuten.

Von den Wirkstoffen der Untergruppe b) sind diejenigen bevorzugt, in denen P für $C_1$–$C_3$-Alkyl, 1,3-Dioxolan-2-ylmethyl und 2,2-Dimethoxyäthyl steht und von den Untergruppe f) diejenigen, in denen A für

$-CH_2-$, 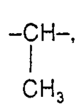, $-CH_2-CH_2-$ oder 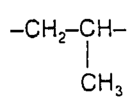 steht

und von der Untergruppe g) diejenigen, in denen $R_1$ Wasserstoff, $C_1$–$C_4$-Alkyl oder $C_2$–$C_4$-Alkenyl bedeutet.

Eine ganz besonders hervorzuhebende Gruppe von Wirkstoffen ist jene, in welchen X für Sauerstoff, P für Methyl und Äthyl, n für 1–3, X–P für Trifluormethyl, 2 für Wasserstoff, A für $-CH_2-$; $-CH_2-CH_2-$; $-CH_2-\underset{CH_3}{CH}-$ und

$X_1$ für Methyl, Äthyl, Propyl, Isopropyl, Isobutyl, sec. Butyl und Allyl und Methoxyiminoäthyl sowie $R_1$ für einen $C_1$–$C_3$Dihalogenalkylrest stehen.

Bevorzugte Einzelverbindungen sind:

N-(3,4-Dimethoxyphenyläthyl)-N-isopropyl-dichloracetamid,
N-(3,4-Dimethoxyphenyläthyl-N-isopropyl-chloracetamid,
N-(3,4-Dimethoxyphenyläthyl)-N-sec.-butyl-dichloracetamid,
N-(3,4-Dimethoxybenzyl)-N-isopropyl-dichloracetamid,
N-(3,4-Dimethoxybenzyl)-N-n-propyl-dichloracetamid,
N-(3,4-Dimethoxybenzyl)-N-(2-methoxyäthyl)-dichloracetamid,
N-(3,4-Dimethoxybenzyl)-N-isopropyl-(2-chlorpropionyl)amid,
N-4-Methoxybenzyl-N-isopropyl-dichloracetamid,
N-4-Trifluormethylbenzyl-N-isopropyl-dichloracetamid,
N-(4-Äthoxy-3-methoxybenzyl)-N-isopropyl-dichloracetamid,
N-(3,4,5-Trimethoxybenzyl)-N-isopropyl-dichloracetamid,
N-[2-(4-Methoxyphenyl)-2-methyl]äthyl-N-isopropyl-dichloracetamid,
N-(3-Methoxybenzyl)-N-isopropyl-dichloracetamid,
N-(2-Methoxybenzyl)-N-isopropyl-dichloracetamid,
N-(2,4-Dimethoxybenzyl)-N-isopropyl-dichloracetamid,
N-(2,5-Dimethoxybenzyl)-N-isopropyl-dichloracetamid,
N-(2,3-Dimethoxybenzyl)-N-isopropyl-dichloracetamid,
N-(3,5-Dimethoxybenzyl)-N-isopropyl-dichloracetamid,
N-[2-(4-Methoxyphenyl)-1-methyl]ethyl-chloracetamid,
N-(4-Methoxyphenyl)-N-(2,2-dimethoxyäthyl)-dichloracetamid,
N-(4-Methoxybenzyl)-N-äthyl-dichloracetamid,
N-(4-Trifluorbenzyl)-chloracetamid,
N-(4-Methoxybenzyl-N-allyloxyiminoäthyl-dichloracetamid,
N-(4-Methoxybenzyl)-N-äthoxyiminoäthyl-dichloracetamid,
N-(4-Methoxybenzyl)-N-methoxyiminoäthyl-dichloracetamid,
N-(3,4-Dimethoxybenzyl)-N-sec.butyl-dichloracetamid,
N-(3,4-Dimethoxybenzyl)-N-isobutyl-dichloracetamid,
N-(3,4-Dimethoxybenzyl)-N-allyl-dichloracetamid,
N-(4-Methoxybenzyl)-dichloracetamid,
N-(3,4,5-Trimethoxyphenyläthyl)-N-isopropyl-dichloracetamid,
N-[4-(1,3-Dioxolan-2-yl-methoxy)benzyl]-dichloracetamid
N-[4-(1,3-Dioxolan-2-yl-methoxy)benzyl]-N-isopropyl-dichloracetamid,
$N_1$-(3,4-Dimethoxybenzyl)-$N_1$-dichloracetyl-$N_2$-äthoxycarbonyl-hydrazid,
N-(3,4-Dimethoxybenzyl)-N-methoxyiminoäthyl-dichloracetamid.

Die Verbindungen der Formel I sind neue Verbindungen mit Ausnahme von N-(4-Methoxybenzyl)-dichloracetamid, welches aus der US-Patentschrift 4 208 203 bekannt ist. Diese Verbindung wird dort unter anderen genannt als Wirkstoff zum Schützen von Maiskulturen vor der phytotoxischen Wirkung von Herbiziden der Thiolcarbamat-Klasse.

Ferner sind N-(3,4-Methylendioxybenzyl)-dichloracetamid und N-Benzyl-N-isopropyldichloracetamid als Wirkstoffe zum Schützen von Nutzpflanzenkulturen gegen Herbizid der Thiolcarbamat-Klasse in der US-Patentschrift 4 137 070 und zum Schützen vor Herbiziden der Chloracetanilid-Klasse in der US-Patentschrift 4 124 376 genannt.

In überraschender und nicht vorhersehbarer Weise schützen die Verbindungen der Formel I, sowie N-(4-Methoxybenzyl)-dichloracetamid auch Sorghum- und Maiskulturen vor der phytotoxischen Wirkung von Herbiziden der Chloracetanilid-Klasse.

Die Acylamide der Formel I werden hergestellt, indem man ein Acylhalid der Formel II

$$R-CO-Q \qquad (II),$$

worin Q Chlor oder Brom oder einen Rest OCOR bedeutet und die unter Formel I gegebene Bedeutung hat, in einem inerten organischen Lösungsmittel, in Gegenwart der mindestens äquimolaren Menge eines säurebindenden Mittels, mit einem Amin der Formel III umsetzt

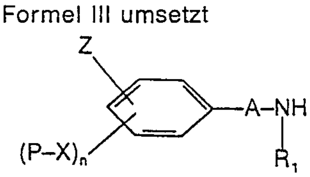

(III),

worin A, n, P, X, $R_1$ und Z die unter Formel I gegebene Bedeutung haben.

Die Reaktion wird zweckmässigerweise in einem reaktionsinerten Lösungsmittel bei Normaldruck durchgeführt. Als Lösungsmittel eignen sich beispielsweise aliphatische oder aromatische Kohlenwasserstoffe wie Benzol, Toluol, Xylole, Cyclohexan, Petroläther, halogenierte Kohlenwasserstoffe wie Chlorbenzol, Methylenchlorid, Äthylenchlorid, Chloroform, Äther und ätherartige Verbindungen wie Diäthyläther, Diisopropyläther, t-Butylmethyläther, Dimethoxyäthan, Dioxan, Tetrahydrofuran, Anisol; Ketone wie Aceton, Methyläthylketon; Ester wie Äthylacetat, Butylacetat und Gemische solcher Lösungsmittel untereinander.

Als säurebindende Mittel sind insbesondere tertiäre Amine wie Trimethylamin, Triäthylamin, Chinuclidin, 1,4-Diazabicyclo-(2,2,2)-octan, 1,5-Diazabicyclo(4,3,0)non-5-en oder 1,5-Diazabicyclo(5,4,0)undec-7-en geeignet. Es können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Ausgangsverbindungen der Formel II sind bekannt; die Ausgangsverbindungen der Formel III sind teilweise bekannt bzw. können nach bekannten Methoden hergestellt werden. Teilweise sind es Pharmaca aus der Reihe der Phenylalkyl- bzw. Alkoxy- oder Dialkoxyphenylakylamine oder Zwischenprodukt zu deren Herstellung und finden Verwendung zur Beeinflussung des vegetativen Nervensystems.

In der EP-A 98 953 sind einige der folgenden Amine der Formel III aufgezählt:
2-(4-Methoxyphenyl)-3-methyl-butylamin,
1,3-Dimethyl-3-methoxyphenyl-butylamin,
1-Methoxyphenyl-äthylamin,
3-Methoxyphenyl-2-methyl-propylamin,
3-Methoxyphenyl-pentylamin,
2-Methoxyphenyl-1-methyläthylamin,
2,3,4-Trimethoxyphenyläthylamin,
3,4-Dimethoxybenzylamin,
N-(3,4-Dimethoxybenzyl)-N-isopropyl-amin,
N-(4-Methoxybenzyl)-N-dimethoxymethyl-amin,
N-(4-Methoxybenzyl)-N-äthyl-amin,
3-(4-Methoxyphenyl)-3-methyl-propyl-amin,
N-(4-Äthoxy-3-methoxybenzyl)-N-isopropyl-amin,
N-(3-Chlor-4-methoxybenzyl)-N-isopropyl-amin,
N-(3,4-Dimethoxybenzyl)-N-n-propyl-amin,
N-(3,4-Dimethoxybenzyl)-N-(2-methoxyäthyl)-amin,
N-(1,1-dimethyl-4-methoxybenzyl)-N-isopropyl-amin,
1,1-Dimethyl-4-methoxy-benzyl-amin,
N-(4-Trifluormethylbenzyl)-N-isopropyl-amin,
N-(3,4-Dimethoxybenzyl)-N-sec.-butyl-amin,
N-(3,4-Dimethoxybenzyl)-N-allyl-amin,
N-(3,4-Dimethoxy-1-methylbenzyl)-N-isopropyl-amin,
3,4-Dimethoxy-1-methylbenzyl-amin,
4-(1,3-Dioxolan-2-yl-methoxy)-benzyl-amin,
N-(4-Methoxybenzyl)-N-äthyl-amin,
N-(1-Methyl-2-m-trifluormethylphenyl-äthyl)-N-äthyl-amin.

Ferner sind in J. Am. Chem. Soc. 95 44 38 (1973) sowie in Tetrahedron Letters 1978 5225 einige der folgenden weiteren Amine der Formel III beschrieben:
3,4-Dimethoxy-styrylamin,
N-(3,4-Dimethoxybenzyl)-N′-äthoxycarbonyl-hydrazid,
N-(3,4-Dimethoxybenzyl)-N-äthoxycarbonylmethyl-amin,
3-Methoxy-4,5-methylendioxybenzyl-amin,
N-[2-(4-Methoxyphenyl)-1-methyl]-N-methyl-amin,
N-(3,4-Dimethoxybenzyl)-N-tetrahydrofurfuryl-amin,
1-Cyano-4-methoxybenzyl-amin,
N-Isopropyl-N-[2-(4-methoxyphenyl)-1-methyl]-amin,
4-Methoxy-1-trifluormethylbenzyl-amin,
N-(3,4-Dimethoxybenzyl)-N-(ααα-trifluoro-isopropyl)-amin,
N-(4-Methoxy-1-methylbenzyl)-N-isopropyl-amin,
N-(3,4-Dimethoxybenzyl)-N-(1-cyanoäthyl)-amin,
2-(3,4-Dimethoxyphenyl)-2-methyl-propylamin,
2,2-Difluoro-2-[4-trifluormethoxyphenyl)-äthyl-amin,
N-(3,4-Dimethoxyphenyläthyl)-N-isopropyl-amin,
N-(3,4-Dimethoxybenzyl)-N-(α-chlorisopropyl)-amin,
N-(4-Diäthoxy-äthoxybenzyl)-N-n-propyl-amin,
N-(4-Diäthoxy-äthoxybenzyl)-N-isopropyl-amin,
N-(4-Diäthoxy-äthoxybenzyl)-N-allyl-amin,
N-[3-(4-Methoxyphenyl)-1-methyl-propyl]-N-isopropyl-amin,
N-[3-(3,4-Dimethoxyphenyl)-1-methylpropyl]-N-isopropyl-amin,
N-[4-(1,3-Dioxolan-2-yl-methoxy)-phenyl]-N-isopropyl-amin,
2-(3,4-Dimethoxyphenyl-2-fluormethyliden-äthyl-amin.

Ein weiteres Verfahren zur Herstellung von Halogenacyl-phenylakylamine der Formel I, in denen $R_1$ eine von Wasserstoff verschiedene Bedeutung hat, besteht darin, daß man ein sekundäres Amin der Formel IIIa

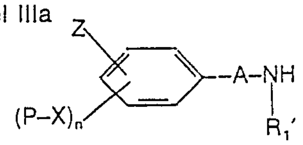

(IIIa),

worin A, n, P, X und Z die unter Formel I gegebene Bedeutung haben während $R_1'$ einen geradkettigen oder verzweigten, gesättigten oder ungesättigten $C_1$–$C_4$-Kohlenwasserstoffrest bedeutet, der unsubstituiert oder durch Alkoxy, Halogen, Cyan oder Trifluormethyl substituiert sein kann, ferner Cycloalkyl, Alkylcycloalkyl, Dialkoxyalkyl, 1,3-Dioxolan-2-ylalkyl, Furylalkyl, Tetrahydrofurylalkyl oder einen Rest $-NHCOOR_2$, $-CH_2COOR_2$ oder $-CH(CH_3)COOR_2$ wobei $R_2$ die unter Formel I gegebene Bedeutung hat, mit Chloral oder einen durch Addition an dessen Oxogruppe gebildetes Chloralderivat in wäßrigem Medium und/oder einem polaren organischen Lösungsmittel, in Gegenwart säurebindender Mittel und der katalytischen Menge eines anorganischen oder organischen Cyanides umsetzt (siehe DE-OS 2 807 340)

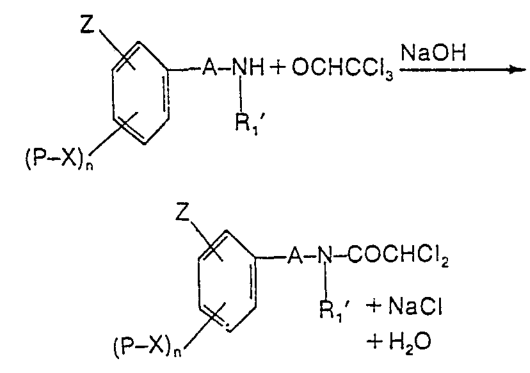

Anstelle von Chloral kann auch Chloralhydrat, anstelle von NaOH auch ein Überschuß an sekundärer Base bzw. einem Alkali- oder Erdalkalisalz einer schwachen Säure wie z. B. Natriumbicarbonat oder Calciumcarbonat verwendet werden. Die Umsetzung wird durch Alkalicyanide beschleunigt.

Ein weiteres Verfahren besteht darin, Phenole bzw. Phenoläther mit einem reaktionsfähigen N-Methylolhalogenalkancarbonsäureamides wie z. B. des Chloressigsäuremethylolamides oder des Dichloressigsäuremethylolamides nach folgender Gleichung umzusetzen.

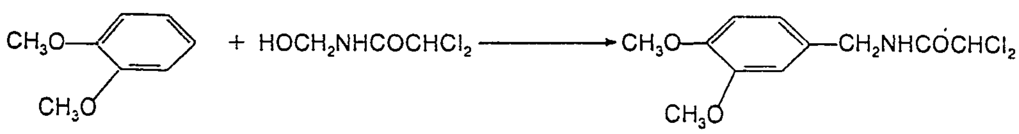

Entsprechend der Struktur der Amine der Formel III sind verschiedene, an sich bekannte Wege zu deren Herstellung nötig.

Wenn das Brückenglied A die Methylengruppe ist, hat man es mit Benzylaminen zu tun, deren Herstellung weitgehend bekannt ist und beispielsweise durch Hydrierung von im Phenylkern entsprechend den Bedeutungen von N, P, X und Z substituierten Benzonitrilen.

Durch katalytische Reduktion von im Phenylkern entsprechend substituierten Acetophenonoximen gelangt man zu α-Methyl-substituierten-Benzylaminen und durch katalytische Hydrierung von entsprechend substituierten α,α,α-Trifluoracetophenonoximen zu in α-Stellung durch die Trifluormethylgruppe substituierten Benzylaminen. Weitere in der α-Stellung substituierte Benzylamine lassen sich durch Anlagerung einer entsprechend substituierten Benzoylverbindung an ein Amin und Erhitzen der Komponenten in Gegenwart von Ameisensäure, Formamid oder Ammoniumformiat in Gegenwart von einer katalytischen Menge einer Lewis Säure (siehe R. Leuckart Ber. 22 1409 und 1851).

Durch Abbau von im Phenylkern und in α-Stellung entsprechend substituierter α,α-Phenylessigsäureamiden mittels eines Alkalihypogenites in wäßrigem Milieu bei ca. 70 °C kann man das Phenylessigsäureamid zum entsprechenden Benzylamin decarboxylieren, (siehe A.W. Hofmann Ber. 18 (1885) 2734).

Durch Hydrierung von im Phenylkern entsprechend den Bedeutungen von n, P, X und Z substituierten Benzylcyaniden lassen sich die entsprechend substituierten primären Phenyläthylamine herstellen. Beispielsweise entsteht aus 3-Trifluormethoxybenzylchlorid (bekannt aus DE-OS 3 228 728) und Alkalicyanid des 3-Trifluormethoxyphenyläthylamin. Sofern das Benzylcyanid in der α-Stellung durch ein oder zwei Alkylreste substituiert ist, entstehen die entsprechend substituierten 2-Aryl-2-alkyl-äthylamine bzw. 2-Aryl-2,2-dialkyl-äthylamine, wie es z. B. die folgende Gleichung darlegt:

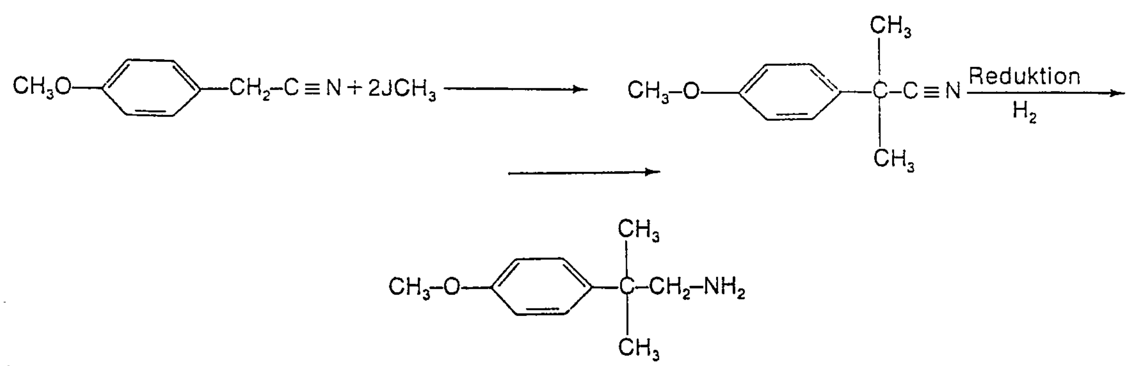


Es können aber auch sekundäre Amine halogenacyliert werden. Diese lassen sich leicht nach an sich bekannten Verfahren z. B. durch Umsetzung eines entsprechend substituierten aromatischen Aldehydes mit einem primären Amin und anschließender Hydrierung herstellen. Durch Kondensation von 3,4-Dimethoxybenzaldehyd und 1-Methyl-2,2,2-trifluoräthylamin und anschließende Hydrierung entsteht das N-(3,4-Dimethoxybenzyl)-N-(1-methyl-2,2,2-trifluoräthyl)-amin gemäß folgendem Schema:

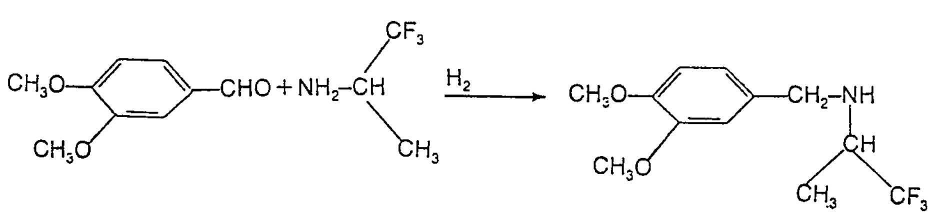

Bei entsprechender Verwendung des 2,2,2-Trifluoräthylamins entsteht das N-(3,4-Dimethoxybenzyl)-N-(2,2,2-trifluoräthyl)amin (siehe DE-OS 3 218 201).

Schiff'sche Basen sind geeignete Grundstoffe zur Herstellung weiterer sekundärer Amine. So entsteht z.B. aus 4-Methoxybenzyliden-butylamin und Isopropylmagnesiumjodid nach folgender Gleichung: (siehe Naturwissenschaften 48 (1961) 129).

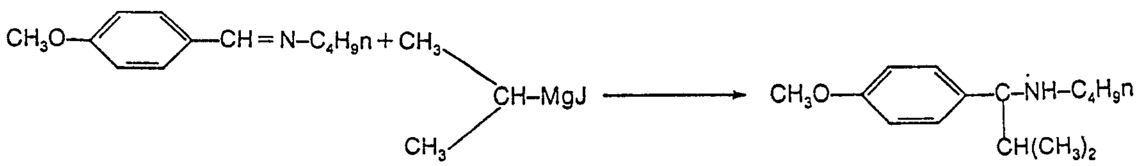

Als weiteres Beispiel für die Herstellung von Aminen der Formel III sei die Anlagerung von Allylmercaptane an Schiff'sche Basen erwähnt, z.B. von 3,4-Dimethoxybenzoliden-allylamin und Methylmercaptan gemäß dem Schema:

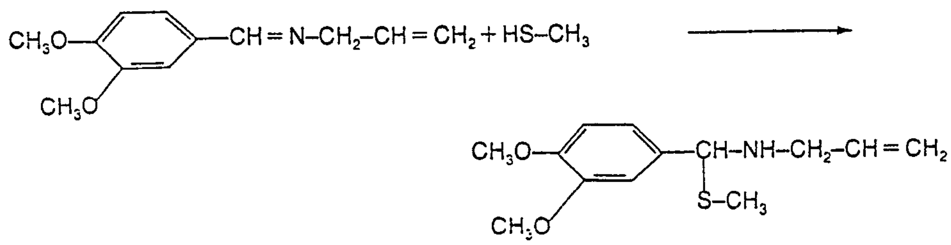

(siehe EP-A 93 610)

Ebenso erhält man durch Umsetzung von Schiff'schen Basen mit Trichloressigsäure nach der folgenden Gleichung z.B. N-(α-Trichlormethyl-benzyl)-N-isopropyl-amin

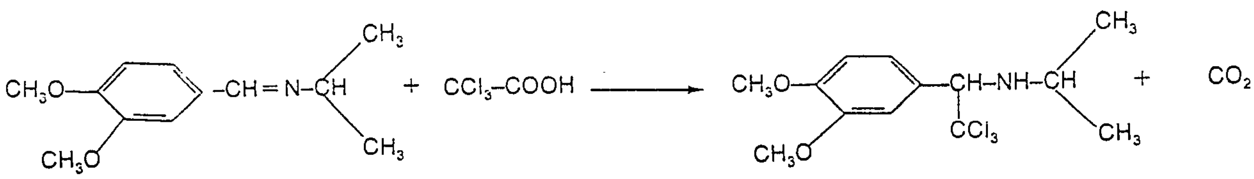

Durch geeignete Reduktion der entsprechend kernsubstituierten Phenylessigsäurealkylamide, z.B. des 3,4-Dimethoxyphenylessigsäure-N-isopropylamides mit Lithiumaluminiumhydrid bzw. Boran entstehen weitere Phenylalkylamine der Formel III

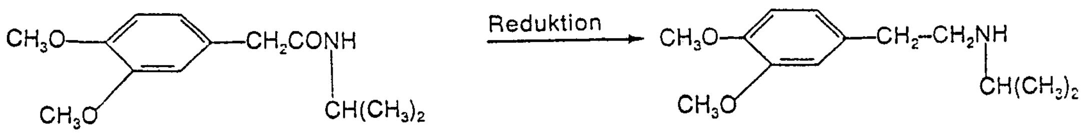


(siehe dazu auch DE-OS 1 959 898)

Durch hydrierende Kondensation von 4-Hydroxybenzylamin (hergestellt nach C.A. 60 (1964) 1627e) mit Phenol, Formaldehyd und Ammoniak in Aceton erhält man in 92%iger Ausbeute 4-Hydroxy-N-isopropyl-benzylamin, welches nach bekannter Methode dichloracetyliert und anschließend methyliert werden kann, gemäß dem Schema:

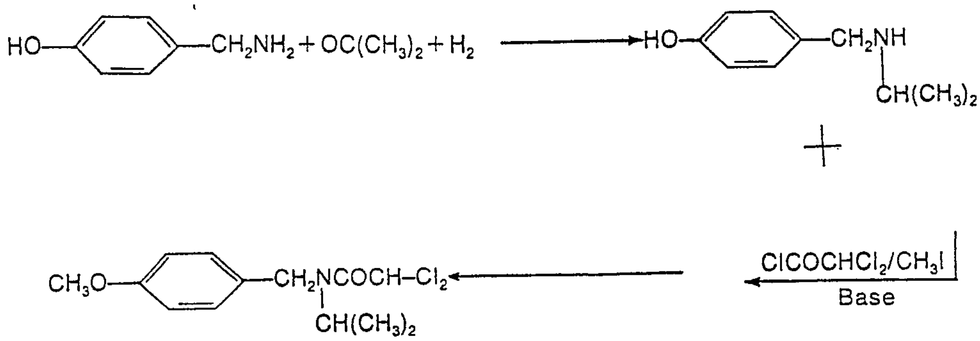

Durch nachträgliche Alkylierung der freien Hydroxylgruppe am Phenylkern kann z.B. 4-Hydroxy-N-allyl-benzyl-dichloramid zu einem Wirkstoff der Formel I umgesetzt werden.

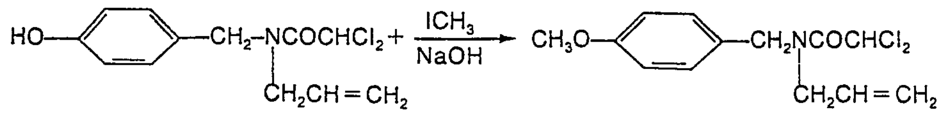


Das p-Allylaminomethylphenol läßt sich leicht herstellen gemäß Z. Heterocycl. Chem. 8 (1971) 605–610.

Verwendet man N-(3,4-Dimethoxybenzyl)-dichloracetamid und Allylchlorid als Ausgangsstoffe, so läßt sich der Verlauf des erfindungsgemäßen Verfahrens durch folgende Gleichung erläutern:

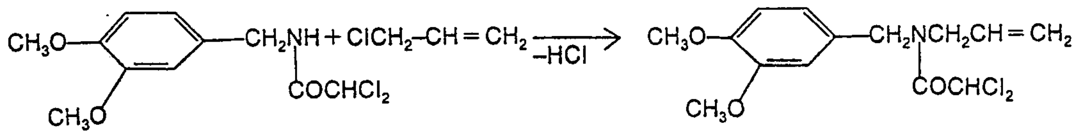


Werden N-Allyl-N-dichloracetamid und 4-Methoxybenzylchlorid als Ausgangsmaterial benutzt, so verläuft das erfindungsgemäße Verfahren gemäß folgender Gleichung

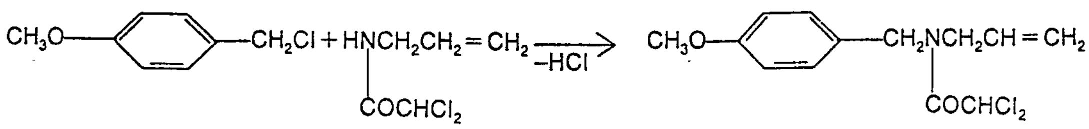


Zur Herstellung von substituierten N-Benzyl-N-alkoxyiminoäthyldichloracetamiden der Formel V

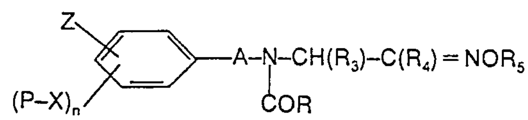
(V)

worin A, n, P, R, $R_3$, $R_4$, $R_5$ und Z die unter Formel I gegebene Bedeutung haben, stehen verschiedene Methoden zur Verfügung.

(a) Umsetzung von reaktionsfähigen Säurederivaten der Formel II

R–COQ (II)

worin Q Chlor, Brom oder einen Rest-OCOR bedeutet und R die unter der Formel I gegebene Bedeutung hat, und einem Amin der Formel VI

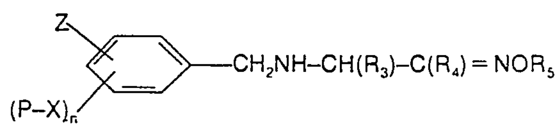
(VI)

worin n, P, $R_3$, $R_4$, $R_5$ und Z die unter Formel I gegebene Bedeutung haben $R_3$ und $R_4$ jedoch bevorzugt Wasserstoff und $R_5$ Methyl, Äthyl oder Allyl bedeutet.

(b) Reaktion eines Halogenacylamines der Formel VII

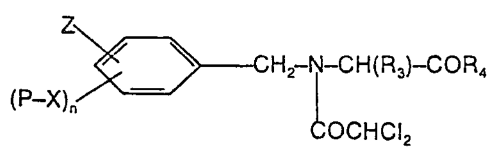
(VII)

worin n, P, $R_3$, $R_4$ und Z die unter der Formel I gegebene Bedeutung haben, mit einem Hydroxylamin der Formel VIII

$H_2N–OR_5 \cdot HCl$ (VIII)

worin $R_5$ die unter Formel I gegebene Bedeutung hat, in Gegenwart eines inerten Lösungs- oder Verdünnungsmittels und eines säurebindenden Mittels.

Die Aminderivate der Formel VI sind neue Verbindungen. Sie lassen sich durch bekannte Verfahren herstellen, wie sie z.B. in der EP-A 35 638 oder der DE-OS 3 004 871 beschrieben sind, durch Umsetzung eines Benzyl Amines der Formel III mit einer Verbindung der Formel VIII

$$Y\text{–}CH(R_3)\text{–}C(R_4)=NOR_5 \qquad (VIII),$$

worin $R_3$, $R_4$ und $R_5$ die unter der Formel I gegebene Bedeutung haben und Y für einen Tosyl- oder Methylsulfonylrest oder ein Halogenatom steht. Die Umsetzung findet in Gegenwart eines inerten Lösungs- oder Verdünnungsmittels und gegebenenfalls eines säurebindenden Mittels statt.

Werden für diese Umsetzung Dichloressigsäurechlorid und 4-Trifluormethylbenzyl-2′-methoxyiminäthylamin als Ausgangsstoffe verwendet, so kann die Reaktion durch die folgende Gleichung dargestellt werden:

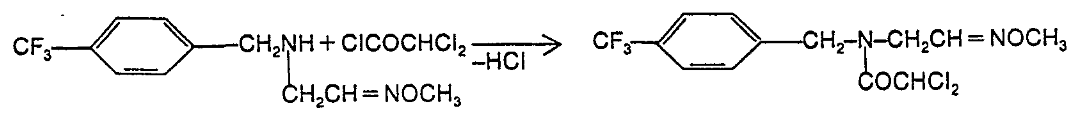


Verwendet man N-(3,4-Dimethoxybenzyl)-N-(2-oxopropyl)-dichloracetamid und O-Methyl-hydroxylamin als Ausgangsmaterialien so verläuft die Umsetzung erfindungsgemäß nach der Gleichung

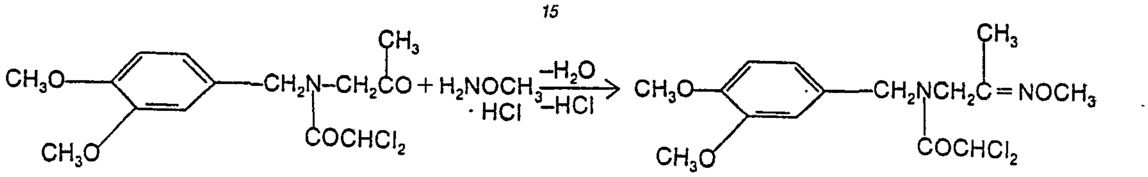


Die Verbindungen der Formel VII lassen sich auf einfache Weise herstellen, indem man ein Keton oder Aldehyd der Formel VIII

$$Y\text{–}CH(R_2)\text{–}C(R_3)=O \qquad (VIII)$$

in welcher $R_2$ und $R_3$ die unter Formel I gegebene Bedeutung haben und Y einen Tosyl- oder Methylsulfonylrest oder ein Halogenatom bedeutet, mit einem Hydroxylamin resp. O-Alkylhydroxylamin Hydrochlorid der Formel IX umsetzt

$$H_2N\text{–}OR_4 \cdot HCl \qquad (IX)$$

worin $R_4$ die unter der Formel I gegebene Bedeutung hat. Diese Umsetzung findet in einem inerten Lösungs- oder Verdünnungsmittel, in Gegenwart eines säurebindenden Mittels, statt.

Das N-(3,4-Dimethoxybenzyl)-N-(1′-methylcarbonyl-äth-1-yl)dichloracetamid läßt sich in einfacher Weise herstellen, indem man
N-(3,4-Dimethoxybenzyl)-N-(1′-methylpropinyl)-dichloracetamid
in saurem Milieu in Gegenwart eines Katalysators mit Wasser behandelt, entsprechend der Gleichung

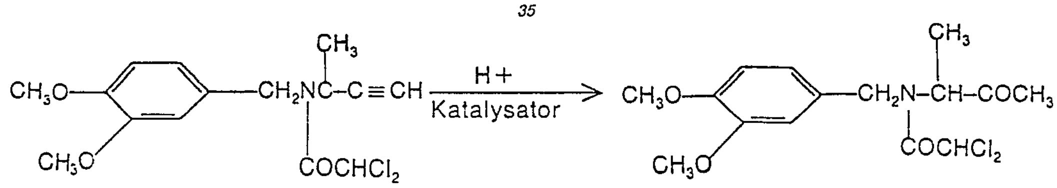


Die Halogenacylamine der Formel VII

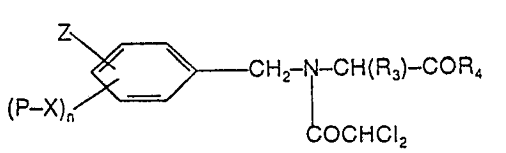 (VII)

worin n, P, $R_3$, $R_4$, X und Z die unter der Formel I gegebene Bedeutung haben, lassen sich über die entsprechenden Acetale der Formel XI herstellen

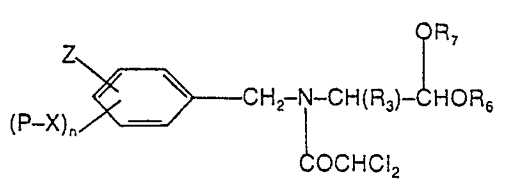 (XI)

worin n, P, $R_3$, X und Z die unter der Formel I gegebene Bedeutung haben und $R_6$ und $R_7$ je $C_1$–$C_6$ Alkyl oder zusammen eine Äthylen- oder Propylenbrücke darstellen, indem man die Acetalreste in einem inerten Lösungs- oder Verdünnungsmittel in Gegenwart eines sauren Katalysators abspaltet. Als saurer Katalysator kommt beispielsweise Schwefelsäure oder Salzsäure in Frage.

Ein Gegenmittel oder Antidote der Formel I kann je nach Anwendungszweck zur Vorbehandlung des Saatgutes der Kulturpflanze (Beizung des Samens oder der Sticklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die preemergente Behandlung schließt sowohl die Behandlung der Anbaufläche vor der Aussaat (ppi = pre plant incorporation) als auch die Behandlung der angesäten,

aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu Herbizid von 1:5 bis 1:50 die volle Schutzwirkung erreicht. Bei der Samenbeizung und ähnlichen gezielten Schutzmaßnahmen werden jedoch weit geringere Mengen Gegenmittel im Vergleich mit den später prp Hektar Anbaufläche verwendeten Mengen an Herbizid benötigt. Im allgemeinen werden bei der Samenbeizung pro kg Samen 0,1–10 g Gegenmittel benötigt. In der Regel wird mit 0,1–5 g Gegenmittel pro kg Samen bereits die volle Schutzwirkung erreicht. Falls das Gegenmittel kurz vor der Aussaat durch Samenquellung appliziert werden soll, so werden zweckmäßig Lösungen des Gegenmittels verwendet, welche den Wirkstoff in einer Konzentration von 1–10 000 ppm enthalten. In der Regel wird mit Konzentrationen des Gegenmittels von 100–1000 ppm die volle Schutzwirkung erreicht.

In der Regel liegt zwischen protektiven Maßnahmen, wie Samenbeizung und Behandlung von Stecklingen mit einem Gegenmittel der Formel I und der möglichen späteren Feldbehandlung mit Agrarchemikalien ein längerer Zeitraum. Vorbehandeltes Saat- und Pflanzengut kann später in der Landwirtschaft, im Gartenbau und in der Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Agrarchemikalien enthalten, vor deren Einfluß die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Öle, Zucker, Stärke, Eiweiß usw., produzieren und zu diesem Zweck angebaut werden. Zu diesen Pflanzen gehören beispielsweise sämtliche Getreidearten, wie Weizen, Roggen, Gerste und Hafer, daneben vor allem Reis, Kulturhirse, Mais, Baumwolle, Zuckerrüben, Zuckerrohr, Soja, Bohnen und Erbsen.

Das Gegenmittel kann überall dort eingesetzt werden, wo eine Kulturpflanze der vorgenannten Art vor der schädlichen Wirkung einer Agrarchemikalie geschützt werden soll. Dabei kommen als Agrarchemikalien in erster Linie Herbizide der verschiedensten Stoffklassen, insbesondere jedoch Halogenacetanilide, in Betracht.

Halogenacetanilide, deren schädigende Wirkung gegenüber Kulturpflanzen mit Hilfe der Acylamid-Derivate der Formel I aufgehoben werden kann, sind bereits in großer Zahl bekannt geworden. Solche Halogenacetaniliden können durch die folgende allgemeine Formel IV beschrieben werden:

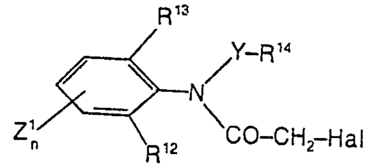

(IV).

In dieser Formel bedeuten Hal Halogen, insbesondere Chlor oder Brom, $R^{12}$ und $R^{13}$ unabhängig voneinander je Wasserstoff, Halogen, niederes Alkyl, Alkoxy, Alkylthio, Halogenalkyl, Alkoxyalkyl oder Alkylthioalkyl, Z'Wasserstoff, Halogen, niederes Alkyl, Alkoxy, Alkylthio, Halogenalkyl, Alkoxyalkyl oder Alkylthioalkyl, wobei die vorgenannten Reste Z vorzugsweise in 3-Stellung in Bezug auf das Stickstoffatom stehen, n 0 bis 3, Y Alkylen, insbesondere Methylen, 1,1- und 1,2-Äthylen, wobei 1,2-Äthylen durch 1–2 niedere Alkylgruppen substituiert sein kann, und $R^{14}$ niedertes Alkoxy, Hydroxycarbonyl, Alkoxycarbonyl, Carbamoyl, N-Alkylcarbamoyl, N,N-Dialkylcarbamoyl, Cyano, ein gegebenenfalls substituierter stickstoffhaltiger heterocyclischer Rest, Alkanoyl, gegebenenfalls substituiertes Benzoyl, gegebenenfalls substituiertes 1,3,4-Oxadiazol-2-yl, 1,3,4-Thiadiazol-2-yl, 1,3,4-Triazol-3-yl oder 1,3,4-Triazol-1-yl bedeutet.

Als einzelne Vertreter solcher Halogenacetanilide seien beispielsweise die folgenden genannt:

N-Äthoxymethyl-N-chloracetyl-2-äthyl-6-methylanilin,
N-Chloracetyl-N-methoxymethyl-2,6-diäthylanilin,
N-Chloracetyl-N-(2-methoxyäthyl)-2,6-dimethylanilin,
N-(2-Allyloxyäthyl)-N-chloracetyl-2,6-dimethylanilin,
N-Chloracetyl-N-(2-n-propoxyäthyl)-2,6-dimethylanilin,
N-Chloracetyl-N-(2-isopropoxyäthyl)-2,6-dimethylanilin,
N-Chloracetyl-N-(2-methoxyäthyl)-2-äthyl-6-methylanilin,
N-Chloracetyl-N-(methoxyäthyl)-2,6-diäthylanilin,
N-(2-Äthoxyäthyl)-N-chloracetyl-2-äthyl-6-methylanilin,
N-Chloracetyl-N-(2-methoxy-1-methyläthyl)-2-methylanilin,
N-Chloracetyl-N-(2-methoxy-1-methyläthyl)-2,6-dimethylanilin,
N-Chloracetyl-N-(2-methoxy-1-methyläthyl)-2,6-diäthylanilin,
N-Chloracetyl-N-(2-methoxy-1-methyläthyl)-2-äthyl-6-methylanilin,
N-(2-Äthoxyäthyl)-N-chloracetyl-2,6-diäthylanilin,
N-Chloracetyl-N-(2-n-propoxyäthyl)-2-äthyl-6-methylanilin,
N-Chloracetyl-N-(2-n-propoxyäthyl)-2,6-diäthylanilin,
N-Chloracetyl-N-(2-isopropoxyäthyl)-2-äthyl-6-methylanilin,
N-Äthyloxycarbonylmethyl-N-chloracetyl-2,6-dimethylanilin,
N-Äthoxycarbonylmethyl-N-chloracetyl-2,6-diäthylanilin,

N-Chloracetyl-N-(2-methoxy-2-methyläthyl)-2,6-dimethylanilin,
N-(2-Äthoxy-2-methyläthyl)-N-chloracetyl-2-äthyl-6-methylanilin,
N-Chloracetyl-N-(1-äthyl-2-methoxyäthyl)-2,6-dimethylanilin,
N-Chloracetyl-N-(2-methoxyäthyl)-2-methoxy-6-methylanilin,
N-n-Butoxymethyl-N-chloracetyl-2-tert.-butylanilin,
N-(2-Äthoxyäthyl-1-methyläthyl)-2,6-dimethylanilin,
N-Chloracetyl-N-(-2-methoxyäthyl)-2-chlor-6-methylanilin,
N-(2-Äthoxyäthyl)-N-chloracetyl-2-chlor-6-methylanilin,
N-(2-Äthoxyäthyl)-N-chloracetyl-2,3,6-trimethylanilin,
N-Chloracetyl-1-(2-methoxyäthyl)-2,3,6-trimethylanilin,
N-Chloracetyl-N-cyanomethyl-2,6-dimethylanilin,
N-But-3-in-1-yl-N-chloracetylanilin,
N-Chloracetyl-N-propargyl-2-äthyl-6-methylanilin,
N-Chloracetyl-N-(1,3-dioxolan-2-ylmethyl)-2,6-dimethylanilin,
N-Chloracetyl-N-(1,3-dioxolan-2-ylmethyl)-2-äthyl-6-methylanilin,
N-Chloracetyl-N-(1,3-dioxan-2-ylmethyl)-2-äthyl-6-methylanilin,
N-Chloracetyl-N-(2-furanylmethyl)-2,6-dimethylanilin,
N-Chloracetyl-N-(2-furanylmethyl)-2-äthyl-6-methylanilin,
N-Chloracetyl-N-(2-tetrahydrofuranylmethyl)-2,6-dimethylanilin,
N-Chloracetyl-N-(N-propargylcarbamoylmethyl)-2,6-dimethylanilin,
N-Chloracetyl-N-(N,N-dimethylcarbamoylmethyl)-2,6-dimethylanilin,
N-(n-Butoxymethyl)-N-chloracetyl-2,6-diäthylanilin,
N-(2-n-Butoxyäthyl)-N-chloracetyl-2,6-diäthylanilin,
N-Chloracetyl-N-(2-methoxy-1,2-dimethyläthyl)-2,6-dimethylanilin,
N-Chloracetyl-N-isopropyl-2,3-dimethylanilin,
N-Chloracetyl-N-isopropyl-2-chloranilin,
N-Chloracetyl-N-(1H-pyrazol-1-ylmethyl)-2,6-dimethylanilin,
N-Chloracetyl-N-(1H-pyrazol-1-ylmethyl)-2-äthyl-6-methylanilin,
N-Chloracetyl-N-(1H-1,2,4-triazol-1-ylmethyl)-2,6-dimethylanilin,
N-Chloracetyl-N-(1H-1,2,4-triazol-1-ylmethyl)-2,6-diäthylanilin,
N-Benzoylmethyl-N-chloracetyl-2,6-dimethylanilin,
N-Benzoylmethyl-N-chloracetyl-2-äthyl-6-methylanilin,
N-Chloracetyl-N-(5-methyl-1,3,4-oxadiazol-2-yl)-2,6-diäthylanilin,
N-Chloracetyl-N-(5-methyl-1,3,4-oxadiazol-2-yl)-2-äthyl-6-methylanilin,
N-Chloracetyl-N-(5-methyl-1,3,4-oxadiazol-2-yl)-2-
N-Chloracetyl-N-methoxycarbonylmethyl-2,6-dimethylanilin,
N-Chloracetyl-N-(2,2-diäthoxyäthyl)-2,6-dimethylanilin,
N-Chloracetyl-N-(2-methoxy-1-methyläthyl)-2,3-dimethylanilin,
N-(2-Äthoxyäthyl)-N-chloracetyl-2-methylanilin,
N-Chloracetyl-N-(2-methoxyäthyl)-2-methylanilin,
tert.butylanilin,
N-Chloracetyl-N-(4-chlorbenzoylmethyl)-2,6-dimethylanilin und
N-Chloracetyl-N-(1-methyl-5-methylthio-1,3,4-triazol-2-ylmethyl)-2,6-diäthylanilin.

Weitere Halogenacetanilide, deren schädigende Wirkung auf Kulturpflanzen durch die neuen Acylamid-Derivate der Formel I aufgehoben werden kann, sind in R. Wegler, Chemie der Pflanzenschutz- und Schädlingsbekämpfungsmittel, Bd. 8, Seiten 90–93 und Seiten 322–327 aufgeführt.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder einem Mittel, welches diese Kombination enthält, behandelt werden.

Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Für die Verwendung von Verbindungen der Formel I oder sie enthaltender Mittel zum Schützen von Kulturpflanzen gegen schädigende Wirkungen von Agrarchemikalien kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäß bis zur gleichmäßigen Verteilung auf der Samenoberfläche (Trockenbeizung). Man verwendet dabei etwa 10 bis 500 g wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Naßbeizung).

c) Beizung durch Tauchen des Saatguts in eine Brühe mit 50–3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10:1 und 1:30) wird verwendet, wobei die Aufwandmenge an Herbizid 0,1 bis 10 kg pro Hektar beträgt. Solche Tankmischung wird vorzugsweise vor oder unmittelbar nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten direkt versprühbaren oder verdünnbaren Lösungen, verdünnten Emulsionen, Spritzpulvern, löslichen Pulvern, Stäubemitteln, Graunulaten, auch Verkapselungen in z.B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Di-octylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnußöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonid oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind z.B. die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxyd-Addukten. Die sulfonierten Benzimidazolderivate enthalten vorzugsweise 2 Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxyd-Adduktes oder Phospholipide in Frage.

Als nichtionisches Tensid kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis

20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi(w-chloräthyl)-äthylammoniumbromid.

Die in der Formulierungstechnik gebräuchlichen Tenside sind u.a. in folgenden Publikationen beschrieben:

«Mc Cutcheon's Detergents and Emulsifiers Annual» MC Pubblishing Corp., Ridgewood, New Jersey, 1981;

H. Stache, «Tensid-Taschenbuch», 2. Auflage, C. Hanser Verlag, München, Wien, 1981;

M. and J. Ash. «Encyclopedia of Surfactants», Vol. I–III, Chemical Publishing Co., New York, 1980–1981.

Die agrochemischen Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermaßen zusammen: (% = Gewichtsprozent)

Emulgierbare Konzentrate

| | |
|---|---|
| Aktiver Wirkstoff | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

Stäube

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

Suspension-Konzentrate

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermaterial: | 5 bis 95%, vorzugsweise 15 bis 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele:

Beispiel 1

Herstellung von N-(3,4-Dimethoxybenzyl)-N-isopropyl-dichloracetanilid

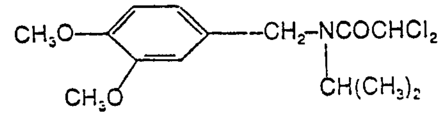

In einem 750 ml Vierhalskolben gibt man 126 g N-(3,4-Dimethoxybenzyl)-N-isopropyl-amin (dargestellt durch hydrierende Kondensation von 3,4-Dimethoxy-benzaldehyd mit Isopropylamin, Kp. 77,5°–78,5°/0,03 mbar) und 250 ml Toluol und rührt bis sich das Amin gelöst hat. Dazu gibt man 120 g 20%ige Natronlauge und rührt unter Kühlen des Kolbens in Alkohol/$CO_2$-Bad bis die Temperatur der Reaktionslösung −10° bis −15 °C beträgt. Dann tropft man langsam in die gerührte Aminlösung eine solche von 89 g Dichloressigsäurechlorid in 100 ml Toluol. Dabei fällt sofort ein weißer Niederschlag aus.

Nachdem alles zugetropft ist, was ca. 1½ Stunde in Anspruch nimmt, wird das Kühlbad weggenommen und das Reaktionsgemisch weitergerührt, bis es Raumtemperatur erreicht. Dann wird auf Eis/Wasser gegossen und die organische Phase im Scheidetrichter mit Toluol extrahiert. Die Toluolphasen werden gesammelt, je zweimal mit 1 N Salzsäure und Wasser gewaschen, getrocknet und eingedampft. Es verbleiben 176 g (91,7% der Theorie) Titelprodukt als helles zähes Öl mit Brechungsindex $n_D^{22}$ 1.5495, welches schließlich erstarrt. Smp. 69–72°.

Beispiel 2

Herstellung von N-(4-Trifluormethylbenzyl)-N-isopropyl-dichloracetamid

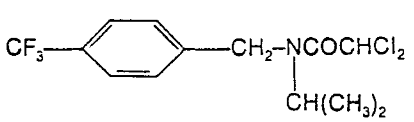

In einem Sulfierkolben wird eine Lösung von 21,7 g N-(4-Trifluormethylbenzyl)-N-isopropylamin vorgelegt und unter Rühren mit 20 ml 20%iger wäßriger Natronlauge versetzt. Das Reaktionsgemisch wird dann unter Rühren mit einem Alkohol/$CO_2$-Bad auf −10° bis −5 °C abgekühlt worauf man dazu langsam 14,7 g Dichloracetylchlorid zutropft. Nach ca. ½ Stunde, wenn alles zugetropft ist nimmt man das Kühlbad weg und rührt weiter noch 2 Stunden bei Raumtemperatur. Dann gibt man das Reaktionsgemisch auf Eis/Wasser und extrahiert die organische Phase mit Toluol. Die Toluolphasen werden gesammelt, erst mit verdünnter Natronlauge, dann mit verdünnter Salzsäure und schließlich zweimal mit Wasser gewaschen, über Natriumsulfat getrocknet und am Rotationsverdampfer eingeengt. Man erhält so ein helles Öl, das bald erstarrt. Die erhaltene Masse wird mit Hexan verrieben und abgenutscht. Man erhält so 21,7 g kristallines Titelprodukt, welches bei 92° bis 93° schmilzt.

Beispiel 3

Herstellung von N-(3,4-Dimethoxyphenyläthyl)-N-isopropyl-dichloracetamid.

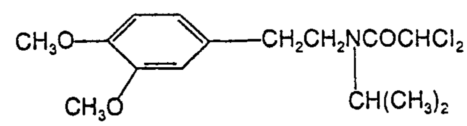

In einem Sulfierkolben wird eine Lösung von 22,3 g N-(3,4-Dimethoxyphenyläthyl)-N-isopropylamin vorgelegt. Dazu gibt man unter Rühren 20 ml 20%ige wäßrige Natronlauge. Dann wird das Reaktionsgemisch mit einem Kühlbad auf eine Temperatur von −10° bis −15° gekühlt, zu der man unter Rühren langsam eine Lösung von 14,7 g Dichloracetylchlorid in 10 ml Toluol tropft. Nachdem alles zugegeben ist, nimmt man das Kühlbad weg und rührt während 2 Stunden bei Raumtemperatur weiter. Dann wird das Reaktionsgemisch auf Eis/Wasser gegossen, mit Toluol extrahiert und die gesammelten organischen Phasen je einmal mit verdünnter Natronlauge, verdünnter Salzsäure und zweimal mit Wasser gewaschen, über Natriumsulfat getrocknet und am Rotationsverdampfer eingedampft. Man erhält so 28,4 g Titelprodukt als dickes Öl.

In analoger Weise zu diesen Beispielen werden folgende Verbindungen hergestellt:

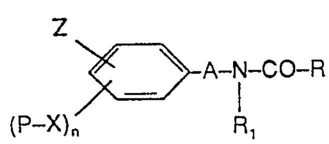 (I),

| No. | Z | $(P{-}X)_n$ | A | $R_1$ | R | phys. Daten |
|---|---|---|---|---|---|---|
| 1 | H | $3,4(CH_3O)_2$ | $CH_2$ | $CH(CH_3)_2$ | $CHCl_2$ | Smp. 69–72° Beispiel 1 |
| 2 | H | $4\text{-}CF_3$ | $CH_2$ | $CH(CH_3)_2$ | $CHCl_2$ | Smp. 82–83° Beispiel 2 |
| 3 | H | $3,4(CH_3O)_2$ | $C_2H_4$ | $CH(CH_3)_2$ | $CHCl_2$ | dickes Öl Beispiel 3 |
| 4 | H | $4\text{-}CH_3O$ | $CH_2$ | H | $CHCl_2$ | Smp. 105–107° |
| 5 | H | $4\text{-}CH_3O$ | $CH_2$ | $CH(CH_3)_2$ | $CHCl_2$ | Sdp. 151–155°/ 0.08 mbar |
| 6 | H | $4\text{-}(CH_3)_2CHO$ | $CH_2$ | H | $CHCl_2$ | Smp. 80–82° |

| No. | Z | (P–X)$_n$ | A | $R_1$ | R | phys. Daten |
|---|---|---|---|---|---|---|
| 7 | H | 4-$(CH_3)_2CHO$ | $CH_2$ | H | $CH_2Cl$ | Smp. 67–68° |
| 8 | H | 4-(1,4-Dioxolan-2-ylmethoxy | $CH_2$ | H | $CHCl_2$ | Smp. 145–148° |
| 9 | H | 3,4$(CH_3O)_2$ | $CH_2$ | H | $CH_2Cl$ | Smp. 117–118° |
| 10 | H | 3,4$(CH_3O)_2$ | $CH_2$ | H | $CHCl_2$ | Smp. 110–112° |
| 11 | H | 4-$CH_3O$ | $CH(CH_3)$ | H | $CH_2Cl$ | Smp. 55–58° |
| 12 | H | 4-$CH_3O$ | $CH_2CH(CH_3)$ | H | $CH_2Cl$ | Smp. 82–84° |
| 13 | H | 4-$CH_3O$ | $CH_2$ | 1,4-Dioxolan-2-yl-methyl | $CHCl_2$ | Öl |
| 14 | H | 4-$CH_3O$ | $CH_2$ | $C_2H_5$ | $CHCl_2$ | Öl |
| 15 | H | 3,4$(CH_3O)_2$ | $CH_2$ | $CH(CH_3)_2$ | $CHCl-CH_3$ | Öl |
| 16 | H | 3,4$(CH_3O)_2$ | $C_2H_4$ | $CH(CH_3)_2$ | $CH_2Cl$ | Öl |
| 17 | H | 3,4$(CH_3O)_2$ | $CH_2$ | $CH(CH_3)C_2H_5$ | $CHCl_2$ | Öl |
| 18 | H | 3,4$(CH_3O)_2$ | $CH_2$ | $C_3H_7$–n | $CHCl_2$ | Öl |
| 19 | H | 3,4$(CH_3O)_2$ | $CH_2$ | $C_2H_4OCH_3$ | $CHCl_2$ | Smp. 80–81° |
| 20 | H | 3,4$(CH_3O)_2$ | $CH_2$ | $CH_2CH=CH_2$ | $CH_2Cl$ | |
| 21 | H | 3,4$(CH_3O)_2$ | $CH_2$ | $CH_2CH=CH_2$ | $CHCl_2$ | |
| 22 | H | 3,4$(CH_3O)_2$ | $C_2H_4$ | $CH_2CH=CH_2$ | $CH_2Cl$ | |
| 23 | H | 3,4$(CH_3O)_2$ | $C_2H_4$ | $CH_2CH=CH_2$ | $CHCl_2$ | |
| 24 | H | 3,4$(CH_3O)_2$ | $CH(CH_3)$ | $CH(CH_3)_2$ | $CH_2Cl$ | |
| 25 | H | 3,4$(CH_3O)_2$ | $CH(CH_3)$ | $CH(CH_3)_2$ | $CHCl_2$ | Öl |
| 26 | H | 4-$CF_3$ | $CH_2$ | H | $CH_2Cl$ | Smp. 95–97° |
| 27 | H | 4-$CF_3$ | $CH_2$ | $CH(CH_3)_2$ | $CH_2Cl$ | Öl |
| 28 | H | 3-$CF_3$ | $CH_2$ | H | $CH_2Cl$ | |
| 29 | H | 3-$CF_3$ | $CH_2$ | H | $CHCl_2$ | |
| 30 | H | 3-$CF_3$ | $CH_2$ | $CH(CH_3)_2$ | $CH_2Cl$ | |
| 31 | H | 3-$CF_3$ | $CH_2$ | $CH(CH_3)_2$ | $CHCl_2$ | |
| 32 | H | 3,4$(CH_3O)_2$ | $CH_2$ | $NH-COOC_2H_5$ | $CHCl_2$ | Smp. 80–83° |
| 33 | H | 3,4$(CH_3O)_2$ | $CH_2$ | Tetrahydro-furan-2-yl | $CHCl_2$ | Smp. 84–86° |
| 34 | H | 4-(1,3-Dioxolan-2-yl-methoxy) | $CH_2$ | $CH(CH_3)_2$ | $CHCl_2$ | Öl |
| 35 | 3-Cl | 4-$CH_3O$ | $CH_2$ | $CH(CH_3)_2$ | $CHCl_2$ | |
| 36 | H | 4-$(CH_3O)_2CHCH_2O$ | $CH_2$ | $CH(CH_3)_2$ | $CHCl_2$ | |
| 37 | H | 3,4$(CH_3O)_2$ | $CH_2$ | $(C_2H_4O)_2C_2H_5$ | $CHCl_2$ | |
| 38 | H | 4-$CH_3O$ | $CH_2$ | Tetrahydro-furan-2-yl | $CHCl_2$ | |
| 39 | H | $N\equiv C-CH_2O$ | $CH_2$ | $CH(CH_3)_2$ | $CHCl_2$ | |
| 40 | H | 4-$CH_3O$ | $CH(CCl_3)$ | $CH(CH_3)_2$ | $CHCl_2$ | |
| 41 | H | $(CH_3O)_2$ | $CH(SCH_3)$ | $CH(CH_3)_3$ | $CHCl_2$ | |
| 42 | H | 3,4$(CH_3O)_2$ | $C(CH_3)_2CH_2$ | $CH(CH_3)_2$ | $CHCl_2$ | |
| 43 | H | 3,4$(CH_3O)_2$ | $CH_2CH(CH_3)$ | $CH(CH_3)_2$ | $CHCl_2$ | |
| 44 | H | 3-$CH_3O$ | $CH_2$ | $CH(CH_3)_2$ | $CHCl_2$ | Öl |
| 45 | H | 2-$CH_3O$ | $CH_2$ | $CH(CH_3)_2$ | $CHCl_2$ | Smp. 81–82° |
| 46 | H | 2,4$(CH_3O)_2$ | $CH_2$ | $CH(CH_3)_2$ | $CHCl_2$ | Smp. 117–118° |
| 47 | H | 2,5$(CH_3O)_2$ | $CH_2$ | $CH(CH_3)_2$ | $CHCl_2$ | Öl |
| 48 | H | 2,3$(CH_3O)_2$ | $CH_2$ | $CH(CH_3)_2$ | $CHCl_2$ | Öl |
| 49 | H | 3,4$(CH_3O)_2$ | $CH_2$ | $CH_2CH(CH_3)_2$ | $CHCl_2$ | Öl |
| 50 | H | 4-$CH_3O$ | $CH_2$ | $CH_2CH(OCH_3)_2$ | $CHCl_2$ | Öl |
| 51 | H | 3,4,5$(CH_3O)_3$ | $CH_2$ | $CH(CH_3)_2$ | $CHCl_2$ | Öl |
| 52 | 3-F | 4-$CH_3O$ | $CH_2$ | $CH(CH_3)_2$ | $CHCl_2$ | |
| 53 | 4-Cl | 2,5$(CH_3O)_2$ | $CH_2CH(CH_3)$ | $CH(CH_3)_2$ | $CHCl_2$ | |
| 54 | 4-$CH_3$ | 2,5$(CH_3O)_3$ | $CH_2CH(CH_3)$ | H | $CHCl_2$ | |
| 55 | 4-CH–$(CH_3)_2$ | 2,5$(CH_3O)_2$ | $CH_2CH(CH_3)$ | H | $CHCl_2$ | |
| 56 | H | 3,4$(CH_3O)_2$ | $C(=CF)CH_2$ | H | $CHCl_2$ | |
| 57 | H | 3,4,5$(CH_3O)_3$ | $CH_2CH_2$ | $CH(CH_3)_2$ | $CHCl_2$ | |
| 58 | 3-$CH_3O$ | 4-$C_2H_5O$ | $CH_2$ | $CH(CH_3)_2$ | $CHCl_2$ | Öl |
| 59 | H | 4-$CH_3O$ | $CH_2CH(CH_2)$ | $CH(CH_3)_2$ | $CHCl_2$ | Öl |
| 60 | H | 2,4$(CH_3O)_2$ | $CH_2$ | $C_3H_7n$ | $CHCl_2$ | Öl |
| 61 | H | 2,4$(CH_3O)_2$ | $CH_2$ | $C_2H_5$ | $CHCl_2$ | |
| 62 | H | 2,4$(CH_3O)_2$ | $CH_2$ | $CH_2CH=CH_2$ | $CHCl_2$ | |
| 63 | H | 2,4$(CH_3O)_2$ | $CH_2$ | $CH_2CH(CH_3)_2$ | $CHCl_2$ | |

| No. | Z | $(P-X)_n$ | A | $R_1$ | R | phys. Daten |
|---|---|---|---|---|---|---|
| 64 | H | $2,4(CH_3O)_2$ | $CH_2$ | $CH(CH_3)C_2H_5$ | $CHCl_2$ | |
| 65 | H | $2,4(CH_3O)_2$ | $CH_2CH_2$ | $CH(CH_3)_2$ | $CHCl_2$ | |
| 66 | H | $4-CH_3-O-CH_2-O$ | $CH_2$ | $CH(CH_3)_2$ | $CHCl_2$ | |
| 67 | $3-CH_3-O-$ | $4-CH_3O-CH_2-O$ | $CH_2$ | $CH(CH_3)_2$ | $CHCl_2$ | |
| 68 | H | $3,4(CH_3-O)_2$ | $CH(CF_3)$ | $CH(CH_3)_2$ | $CHCl_2$ | |
| 69 | H | $3-CH_3-O$ | CH(CN) | H | $CHCl_2$ | |
| 70 | H | $4-CH_3-O$ | $CH(OCH_3)CH_2$ | $CH(CH_3)_2$ | $CHCl_2$ | |
| 71 | H | $3,4(CH_3-O)_2$ | $CH(OCH_3)CH_2$ | $CH(CH_3)_2$ | $CHCl_2$ | |
| 72 | H | $4-CF_3-O$ | $C_2H_4$ | $CH_2-CH_3$ | $CHCl_2$ | |
| 73 | H | $3-CF_3-O-$ | $C_2H_4$ | $CH(CH_3)_2$ | $CHCl_2$ | |
| 74 | H | $4-CF_3-S$ | $C_2H_4$ | $CH(CH_3)_2$ | $CHCl_2$ | |
| 75 | H | $4-ClF_2C-O$ | $C_2H_4$ | $CH(CH_3)_2$ | $CHCl_2$ | |
| 76 | H | $4-F_2HC-O$ | $C_2H_4$ | $CH(CH_3)_2$ | $CHCl_2$ | |
| 77 | $3-C_2H_5-O$ | $4-C_2H_5-O$ | $CH_2$ | $CH(CH_3)_2$ | $CHCl_2$ | |
| 78 | H | $3,4-(H_3C)_2$ | CH(CN) | H | $CHCl_2$ | |
| 79 | H | $4-Cl_2FC-O$ | $CH_3-CH_2$ | $CH(CH_3)_2$ | $CHCl_2$ | |
| 80 | H | $4-CH_3-O$ | CH(CN) | $CH(CH_3)_2$ | $CHCl_2$ | |
| 81 | $3-CH_3$ | $4-CH_3-O$ | $CH_2$ | $CH(CH_3)_2$ | $CHCl_2$ | |
| 82 | $3-CH_3$ | $4-CH_3-O$ | $C_2H_4$ | $CH(CH_3)$ | $CHCl_2$ | |
| 83 | $3-CH_3$ | $4-C_2H_5-O$ | $CH_2$ | $CHCH(CH_3)_2$ | $CHCl_2$ | |
| 84 | $3-CH_3-O$ | $4-CHF_2-O$ | $CH_2$ | $CH(CH_3)$ | $CHCl_2$ | |
| 85 | $3-CH_3-O$ | $4-CH_2-F-O$ | $CH_2$ | $CH(CH_3)_2$ | $CHCl_2$ | |
| 86 | $3-CH_3-O$ | $4-CHFCl-CF_2-O$ | $CH_2$ | $CH(CH_3)_2$ | $CHCl_2$ | |
| 87 | $3-CH_3-O$ | $4-CH_3=CH-CH_2-O$ | $CH_2$ | $CH(CH_3)_2$ | $CHCl_2$ | |
| 88 | $3-CH_3-O$ | $4-N\equiv C-CH_3$ | $CH_2$ | $CH(CH_3)_2$ | $CHCl_2$ | |
| 89 | H | $3,4-(CH_3-O)_2$ | $CH_2$ | $CH(CH_3)CH_2OCH_3$ | $CHCl_2$ | |
| 90 | H | $3,4(CH_3-O)_2$ | $C_2H_4$ | $CH(CH_3)CH_2OCH_3$ | $CHCl_2$ | |
| 91 | H | $3,4(CH_5O)_2$ | $CH_2$ | $CH(CH_3)_2$ | $CH_2CH_2Cl$ | |
| 92 | $4-CH_3O$ | $3-C_2H_5O$ | $CH_2$ | $CH(CH_3)_2$ | $CHCl_2$ | |
| 93 | H | $4-CH_3O$ | $CH_2$ | $CH_2CH=CH_2$ | $CHCl_2$ | |
| 94 | H | $3,4(CH_3O)_2$ | $C_2H_4$ | $CH_2C(CH_3)=NOCH_3$ | $CHCl_2$ | |
| 95 | H | $3,4(CH_3O)_2$ | $CH_2$ | $CH_2C(CH_3)=NOCH_3$ | $CHCl_2$ | |
| 96 | H | $3,4(CH_3O)_2$ | $CH_2$ | $CH(CH_3)CH=NOCH_3$ | $CHCl_2$ | |
| 97 | H | $3,4(CH_3O)_2$ | $CH_2$ | $CH_2CH=NOC_2H_5$ | $CHCl_2$ | |
| 98 | H | $3,4(CH_3O)_2$ | $CH_2$ | $CH_2C(CH_3)=NOCH_3$ | $CHCl_2$ | |
| 99 | H | $4-CH_3O$ | $CH_2$ | $CH_2CH=NOC_2H_5$ | $CHCl_2$ | |

Formulierungsbeispiele für Wirkstoffe der Formel I oder Mischungen dieser Wirkstoffe mit Herbiziden

Beispiel 4

| Spritzpulver | a) | b) | c) |
|---|---|---|---|
| Wirkstoff der Formel I oder Mischung mit Herbizid | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthlenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Beispiel 5

| Emulsion-Konzentrat | a) | b) |
|---|---|---|
| Wirkstoff der Formel I oder Mischung mit Herbizid | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

Beispiel 6

| Stäubemittel | a) | b) |
|---|---|---|
| Wirkstoff der Formel I oder Mischung mit Herbizid | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

Beispiel 7

| Extruder Granulat | a) | b) |
|---|---|---|
| Wirkstoff der Formel I oder Mischung mit Herbizid | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschließend im Luftstrom getrocknet.

Beispiel 8

| Umhüllungs-Granulat | |
|---|---|
| Wirkstoff der Formel I oder Mischung mit Herbizid | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

Beispiel 9

| Suspensions-Konzentrat | a) | b) |
|---|---|---|
| Wirkstoff der Formel I oder Mischung mit Herbizid | 40% | 5% |
| Äthylenglykol | 10% | 10% |
| Nonylphenolpolyäthylenglykoläther (15 Mol) AeO) | 6% | 1% |
| Na-Ligninsulfonat | 10% | 5% |
| Carboxymethylcellulose | 1% | 1% |
| 37%-ige wässrige Formaldehyd-Lösung | 0,2% | 0,2% |
| Silikonöl in Form einer 75%-igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 10

| Salzlösung | |
|---|---|
| Wirkstoff der Formel I oder Mischung mit Herbizid | 5% |
| Isopropylamin | 1% |
| Octylphenolpolyäthylenglykoläther (78 Mol AeO) | 3% |
| Wasser | 91% |

Biologische Beispiele

Die Fähigkeit der Verbindungen der Formel I, Kulturpflanzen vor der phytotoxischen Wirkung starker Herbizide zu schützen, kann aus dem folgenden Beispiel ersehen werden. In der Versuchsbeschreibung werden die Verbindungen der Formel I als Safener oder Gegenmittel (Antidote) bezeichnet.

Beispiel 11

Versuch mit Herbizid und Gegenmittel in Mais.

Herbizid und Gegenmittel werden zusammen als Tankmischung im Vorauflaufverfahren appliziert.

Plastikcontainer (25 cm lang × 17 cm breit × 12 cm hoch) werden mit sandiger Lehmerde gefüllt und Maissamen der Sorte LG 5 eingesät. Nach dem Bedecken der Samen wird die als Safener zu prüfende Substanz zusammen mit dem Herbizid in verdünnter Lösung als Tankmischung auf die Bodenoberfläche gesprüht. 21 Tage nach der Applikation wird die Schutzwirkung des Safeners in Prozent ausgewertet. Als Referenz dienen dabei die mit dem Herbizid allein behandelten Pflanzen (keine Schutzwirkung) sowie die vollständig unbehandelte Kontrolle. (100%ige Schutzwirkung)

Herbizid: N-Chloracetyl-N-(2-methoxy-1-methyl-äthyl)-2-äthyl-6-methyl-anilid (Metolachlor)

| Gegenmittel Verbindung No. | Aufwandmenge | Herbizid Aufwandmenge | relative Schutzwirkung |
|---|---|---|---|
| 1 | 1 kg/ha | 4 kg/ha | 75% |
| 2 | 1 kg/ha | 4 kg/ha | 75% |
| 3 | 1 kg/ha | 4 kg/ha | 75% |
| 4 | 1 kg/ha | 4 kg/ha | 75% |
| 5 | 1 kg/ha | 4 kg/ha | 75% |
| 8 | 1 kg/ha | 4 kg/ha | 63% |
| 12 | 1 kg/ha | 4 kg/ha | 75% |
| 14 | 1 kg/ha | 4 kg/ha | 60% |
| 15 | 1 kg/ha | 4 kg/ha | 38% |
| 16 | 1 kg/ha | 4 kg/ha | 38% |
| 17 | 1 kg/ha | 4 kg/ha | 75% |
| 18 | 1 kg/ha | 4 kg/ha | 63% |
| 19 | 1 kg/ha | 4 kg/ha | 50% |
| 26 | 1 kg/ha | 4 kg/ha | 63% |
| 32 | 1 kg/ha | 4 kg/ha | 63% |
| 34 | 1 kg/ha | 4 kg/ha | 63% |
| 37 | 1 kg/ha | 4 kg/ha | 50% |
| 44 | 1 kg/ha | 4 kg/ha | 50% |
| 45 | 1 kg/ha | 4 kg/ha | 50% |
| 46 | 1 kg/ha | 4 kg/ha | 63% |
| 49 | 1 kg/ha | 4 kg/ha | 63% |
| 50 | 1 kg/ha | 4 kg/ha | 63% |
| 51 | 1 kg/ha | 4 kg/ha | 75% |
| 58 | 1 kg/ha | 4 kg/ha | 75% |

Beispiel 12
Versuch mit Antidote und Herbizid in Hirse im Vorauflaufverfahren. Applikation der Antidote durch Samenbeizung.

Hirsesamen der Sorte Funk G 522 werden mit dem Antidote in einem Glaskolben zusammengegeben. Samen und Produkt werden durch Schütteln und Rotation des Glaskolbens gut durchgemischt. Anschließend wird der so gebeizte Samen in einen mit sandiger Erde gefüllten Plastikcontainer (25 × 17 $cm^2$ Bodenfläche, 12 cm hoch) eingesät. Der Samen wird mit einer dünnen Erdschicht bedeckt. Darauf wird nun eine wäßrige Herbizidemulsion in der gewünschten Applikationsmenge gesprüht. Der Zustand der Pflanzen wird 21 Tage nach der Behandlung evakuiert und die Schutzwirkung des Gegenmittels in Prozent ausgewertet. Als Referenz dienten dabei die mit dem Herbizid allein behandelten Pflanzen (keine Schutzwirkung) sowie unbehandelte Kontrollpflanzen (nochmals Wachstum = 100%ige Schutzwirkung).

Herbizid: N-Chloracetyl-N-(2-methoxy-1-methyläthyl)-2-äthyl-6-methyl-anilid (Metolachlor)

| Gegenmittel Verbindung No. | Aufwandmenge | Herbizid Aufwandmenge | relative Schutzwirkung |
|---|---|---|---|
| 2 | 0,5 kg/ha | 2 kg/ha | 38% |
| 15 | 0,5 kg/ha | 2 kg/ha | 38% |
| 16 | 0,5 kg/ha | 2 kg/ha | 38% |

**Patentansprüche**

1. Ein Mittel zum Schützen von Kulturpflanzen vor der phytotoxischen Wirkung von herbizid wirksamen Chloracetaniliden, dadurch gekennzeichnet, daß es neben inertem Trägermaterial ein Chloracetanilid-Herbizid und als antagonisierenden Wirkstoff ein Acylamid-Derivat der Formel I

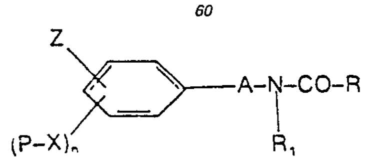

worin

X O oder S, SO, $SO_2$,

P Alkyl, Alkenyl, Alkinyl, Cycloalkyl, Alkoxyalkyl, Alkenoxyalkyl, Alkinoxyalkyl, Alkylthioalkyl, Alkenylthioalkyl, Alkinylthioalkyl, Alkylsulfinylalkyl, Alkylsulfonylalkyl, Halogenalkyl, Cyanalkyl, 2,2-Dialkoxy-alkyl, 1,3-Dioxolan-2-ylalkyl, 1,3-Dioxolan-4-ylalkyl, 2,2-Dialkyl-1,3-dioxolan-4-ylalkyl, 1,3-dioxan-yl-2-alkyl, 2-Benzpyranyl-alkyl, Alkoxycarbonyl oder Alkenyloxycarbonyl oder Tetrahydrofurfurylalkyl, die Gruppe

P–X auch Halogenalkyl

n 1–3

Z Wasserstoff, Alkyl, Halogen, Dioxymethylen, Alkoxy, Alkenyloxy, Alkinyloxy oder Cyanoalkyl

A einen geradkettigen, verzweigten oder cyclischen $C_1$–$C_8$-Kohlenwasserstoffrest, welcher unsubstituiert oder durch Alkoxy, Alkylthio, Fluor, Cyan oder Halogenalkyl substituiert sein kann,

R Halogenalkyl oder Halogenalkenyl,

$R_1$ Wasserstoff, einen geradkettigen oder verzweigten, gesättigten oder ungesättigten $C_1$–$C_5$-Kohlenwasserstoffrest der unsubstituiert oder durch Alkoxy, poly-Alkoxy, Halogen, Cyan oder Trifluormethyl, substituiert sein kann, oder Cycloalkyl, Alkylcycloalkyl, Dialkoxyalkyl, 1,3-Dioxolan-2-ylalkyl, 1,3-Dioxolan-4-alkyl, 1,3-Dioxan-2-ylalkyl, Furylalkyl, Tetrahydrofurylalkyl oder einen Rest $-NHCOOR_2$, $-CH_2COOR_2$, $-CH(CH_3)COOR_2$ oder einen Alkoxyimino-alkylrest$-CH(R_3)-C(R_4)=NOR_5$, wobei

$R_2$ Methyl, Äthyl, Propyl, Isopropyl oder Allyl,

$R_3$ und $R_4$ je Wasserstoff oder $C_1$–$C_4$-Alkyl und

$R_5$ Wasserstoff, $C_1$–$C_4$-Alkyl, $C_3$–$C_4$-Alkenyl oder $C_3$–$C_4$-Alkinyl bedeuten.

2. Mittel gemäß Anspruch 1, dadurch gekennzeichnet, daß im Acylamid-Derivat der Formel I X für Sauerstoff steht.

3. Mittel gemäß Anspruch 1, dadurch gekennzeichnet, daß im Acylamid-Derivat der Formel I R für $C_1$–$C_6$-Halogenalkyl oder $C_2$–$C_6$-Halogenalkylen steht.

4. Mittel gemäß Anspruch 3, dadurch gekennzeichnet, daß im Acylamid-Derivat der Formel I R für $C_1$–$C_6$-Halogenalkyl steht.

5. Mittel gemäß Anspruch 1, dadurch gekennzeichnet, daß im Acylamid-Derivat der Formel I n 1, 2 oder 3 bedeutet.

6. Mittel gemäß Anspruch 1, dadurch gekennzeichnet, daß im Acylamid-Derivat der Formel I P–X 4-Methoxy bedeutet.

7. Mittel gemäß Anspruch 1, dadurch gekennzeichnet, daß im Acylamid-Derivat der Formel I P–X 3,4-Dimethoxy bedeutet.

8. Mittel gemäß Anspruch 1, dadurch gekennzeichnet, daß im Acylamid-Derivat der Formel I P–X Trifluormethyl bedeutet.

9. Mittel gemäß Anspruch 1, dadurch gekennzeichnet, daß im Acylamid-Derivat der Formel I P–X 4-(2,2-Dimethoxy)alkyl bedeutet.

10. Mittel gemäß Anspruch 1, dadurch gekennzeichnet, daß im Acylamid-Derivat der Formel I P–X 4-(1,3-Dioxolan-2-yl)alkyl bedeutet.

11. Mittel gemäß Anspruch 1, dadurch gekennzeichnet, daß A für $C_1$–$C_8$-Alkylen steht.

12. Mittel gemäß Anspruch 1, dadurch gekennzeichnet, daß A für $C_1$–$C_3$-Alkylen steht.

13. Mittel gemäß Anspruch 1, dadurch gekennzeichnet, daß $R_1$ für Wasserstoff steht.

14. Mittel gemäß Anspruch 1, dadurch gekennzeichnet, daß $R_1$ für $C_1$–$C_6$-Alkyl oder $C_2$–$C_4$-Alkylen steht.

15. Mittel gemäß Anspruch 1, dadurch gekennzeichnet, daß $R_1$ für $C_1$–$C_4$-Alkoxy steht.

16. Mittel gemäß Anspruch 1, dadurch gekennzeichnet, daß es als Acylamid-Derivat N-(3,4-Dimethoxybenzyl)-N-isopropyl-dichloracetamid enthält.

17. Mittel gemäß Anspruch 1, dadurch gekennzeichnet, daß es als Acylamid-Derivat N-(3,4-Dimethoxyphenyläthyl)-N-isopropyl-dichloracetamid enthält.

18. Mittel gemäß Anspruch 1, dadurch gekennzeichnet, daß es als Acylamid-Derivat N-(4-Trifluormethylbenzyl)-N-isopropyl-dichloracetamid enthält.

19. Mittel gemäß Anspruch 1, dadurch gekennzeichnet, daß es als Acylamid-Derivat N-(3,4-Dimethoxybenzyl)-N-isobutyl-dichloracetamid enthält.

20. Mittel gemäß Anspruch 1, dadurch gekennzeichnet, daß es als Acylamid-Derivat N-(3,4-Dimethoxybenzyl)-N-n-propyl-dichloracetamid enthält.

21. Mittel gemäß Anspruch 1, dadurch gekennzeichnet, daß es als Acylamid-Derivat N-(3,4-Dimethoxyphenyläthyl)-N-n-propyl-dichloracetamid enthält.

22. Mittel gemäß Anspruch 1, dadurch gekennzeichnet, daß es als Acylamid-Derivat N-(3,4-Dimethoxybenzyl)-N-sec.butyl-dichloracetamid enthält.

23. Mittel gemäß Anspruch 1, dadurch gekennzeichnet, daß es als Acylamid-Derivat N-(3,4-Dimethoxybenzyl)-N-allyl-dichloracetamid enthält.

24. Mittel gemäß Anspruch 1, dadurch gekennzeichnet, daß es als Acylamid-Derivat N-(4-Äthoxy-3-methoxybenzyl)-N-isopropyl-dichloracetamid enthält.

25. Mittel gemäß Anspruch 1, dadurch gekennzeichnet, daß es als Acylamid-Derivat N-(3,4-Dimethoxybenzyl)-N-(2-methoxyäthyl)-dichloracetamid enthält.

26. Mittel gemäß Anspruch 1, dadurch gekennzeichnet, daß es als Acylamid-Derivat N-(3,4-Dimethoxybenzyl)-N-isopropyl-(2-chlorpropionyl)amid enthält.

27. Mittel gemäß Anspruch 1, dadurch gekennzeichnet, daß es als Acylamid-Derivat

N-(4-Methoxybenzyl)-N-isopropyl-dichloracetamid enthält.

28. Mittel gemäß Anspruch 1, dadurch gekennzeichnet, daß es als Acylamid-Derivat N-(3,4,5-Trimethoxybenzyl)-N-isopropyl-dichloracetamid enthält.

29. Mittel gemäß Anspruch 1, dadurch gekennzeichnet, daß es als Acylamid-Derivat N-(3,4-Dimethoxybenzyl)-N-isopropyl-chloracetamid enthält.

30. Mittel gemäß Anspruch 1, dadurch gekennzeichnet, daß es als Acylamid-Derivat N-[2-(4-Methoxybenzyl)-2-methyl]äthyl-N-isopropyl-dichloracetamid enthält.

31. Mittel gemäß Anspruch 1, dadurch gekennzeichnet, daß es als Acylamid-Derivat $N_1$-(3,4-Dimethoxybenzyl)-$N_1$-dichloracetyl-$N_2$-äthoxycarbonyl-hydrazid enthält.

32. Mittel gemäß Anspruch 1, dadurch gekennzeichnet, daß es als Acylamid-Derivat N-(3-Methoxybenzyl)-N-isopropyl-dichloracetamid enthält.

33. Mittel gemäß Anspruch 1, dadurch gekennzeichnet, daß es als Acylamid-Derivat N-(2-Methoxybenzyl)-N-isopropyl-dichloracetamid enthält.

34. Mittel gemäß Anspruch 1, dadurch gekennzeichnet, daß es als Acylamid-Derivat N-(2,4-Dimethoxybenzyl)-N-isopropyl-dichloracetamid enthält.

35. Mittel gemäß Anspruch 1, dadurch gekennzeichnet, daß es als Acylamid-Derivat N-(2,5-Dimethoxybenzyl)-N-isopropyl-dichloracetamid enthält.

36. Mittel gemäß Anspruch 1, dadurch gekennzeichnet, daß es als Acylamid-Derivat N-(2,3-Dimethoxybenzyl)-N-isopropyl-dichloracetamid enthält.

37. Mittel gemäß Anspruch 1, dadurch gekennzeichnet, daß es als Acylamid-Derivat N-(2,4-Dimethoxybenzyl)-N-isopropyl-dichloracetamid enthält.

38. Mittel gemäß Anspruch 1, dadurch gekennzeichnet, daß es als Acylamid-Derivat N-[2-(4-Methoxybenzyl)-2-methyl]äthyl-chloracetamid enthält.

39. Mittel gemäß Anspruch 1, dadurch gekennzeichnet, daß es als Acylamid-Derivat N-(4-Methoxybenzyl)-N-(2,2-dimethoxyäthyl)-dichloracetamid enthält.

40. Mittel gemäß Anspruch 1, dadurch gekennzeichnet, daß es als Acylamid-Derivat N-(4-Methoxybenzyl)-N-äthyl-dichloracetamid enthält.

41. Mittel gemäß Anspruch 1, dadurch gekennzeichnet, daß es als Acylamid-Derivat N-4-Trifluormethylbenzyl-chloracetamid enthält.

42. Mittel gemäß Anspruch 1, dadurch gekennzeichnet, daß es als Acylamid-Derivat N-(3,4,5-Trimethoxyphenyläthyl)-N-isopropyl-dichloracetamid enthält.

43. Mittel gemäß Anspruch 1, dadurch gekennzeichnet, daß es als Acylamid-Derivat N-[4-(1,3-Dioxolan-2-yl-methoxy)benzyl]-dichloracetamid enthält.

44. Mittel gemäß Anspruch 1, dadurch gekennzeichnet, daß es als Acylamid-Derivat N-[4-(1,3-Dioxolan-2-yl-methoxy)-benzyl]-N-isopropyl-dichloracetamid enthält.

45. Mittel gemäß Anspruch 1, dadurch gekennzeichnet, daß es als Acylamid-Derivat N-(3,4-Dimethoxybenzyl)-N-methoxyiminoäthyl-dichloracetamid enthält.

46. Mittel gemäß Anspruch 1, dadurch gekennzeichnet, daß es als Acylamid-Derivat N-(3,4-Dimethoxybenzyl)-N-äthoxyiminoäthyl-dichloracetamid enthält.

47. Mittel gemäß Anspruch 1, dadurch gekennzeichnet, daß es als Acylamid-Derivat N-(3,4-Dimethoxybenzyl)-N-methoxyiminoisopropyl-dichloracetamid enthält.

48. Mittel gemäß Anspruch 1, dadurch gekennzeichnet, daß es als Acylamid-Derivat N-(4-Trifluormethylbenzyl)-N-methoxyiminoäthyl-dichloracetamid enthält.

49. Verfahren zur selektiven Bekämpfung von Unkräutern in Nutzpflanzenkulturen, dadurch gekennzeichnet, daß man die Kulturpflanzen oder deren Anbaufläche sowohl mit einem herbizid wirksamen Chloracetanilid, als auch einer wirksamen Menge eines Acylamid-Derivates der Formel I gemäß Anspruch 1 als Gegenmittel behandelt.

50. Verwendung der Acylamid-Derivate der Formel I gemäß Anspruch 1 zum Schützen von Kulturpflanzen gegen die schädigende Wirkung von herbizid wirksamen Chloracetaniliden.

51. Verfahren zum Schützen von Kulturpflanzen vor Schäden, die bei der Applikation von Chloracetanilid-Herbiziden auftreten, dadurch gekennzeichnet, daß man entweder die Anbaufläche für die Pflanze vor oder während der Applikation des Herbizids oder den Samen oder die Stecklinge der Pflanzen oder die Pflanze selbst mit einer wirksamen Menge eines Acylamid-Derivat der Formel I gemäß Anspruch 1 behandelt.

52. Saatgut von Nutzpflanzen, das sowohl mit einem Chloracetanilid-Herbizid, als auch mit einer antagonistisch wirksamen Menge eines Acyl-

amid-Derivates der Formel I gemäß Anspruch 1 behandelt worden ist.

## Claims

1. An agent for protecting crop plants from the phytotoxic action of herbicidally active chloroacetanilides, which contains in addition to an inert carrier material a chloroacetanilide herbicide and as the antagonistic active substance an acylamide derivative of the formula I

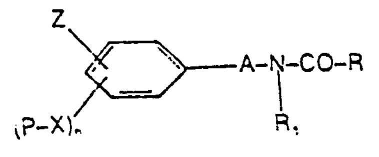

in which

X is O, S, SO or $SO_2$,

P is alkyl, alkenyl, alkinyl, cycloalkyl, alkoxyalkyl, alkenoxyalkyl, alkinoxyalkyl, alkylthioalkyl, alkenylthioalkyl, alkinylthioalkyl, alkylsulfinylalkyl, alkylsulfonylalkyl, halogenoalkyl, cyanoalkyl, 2,2-dialkoxyalkyl, 1,3-dioxolan-2-ylalkyl, 1,3-dioxolan-4-ylalkyl, 2,2-dialkyl-1,3-dioxolan-4-ylalkyl, 1,3-dioxan-2-ylalkyl, 2-benzopyranylalkyl, alkoxycarbonyl, alkenyloxycarbonyl or tetrahydrofurfurylalkyl, the group

P–X also is halogenalkyl;

n is 1–3,

Z is hydrogen, alkyl, halogen, dioxymethylene, alkoxy, alkenyloxy, alkinyloxy or cyanoalkyl,

A is a $C_1$–$C_8$hydrocarbon radical which may be straight-chain, branched or cyclic and which can be unsubstituted or substituted by alkoxy, alkylthio, fluorine, cyano or halogenoalkyl,

R is halogenoalkyl or halogenoalkenyl,

$R_1$ is hydrogen, a $C_1$–$C_5$hydrocarbon radical which is straight-chain or branched, saturated or unsaturated and can be unsubstituted or substituted by alkoxy, polyalkoxy, halogen, cyano or trifluoromethyl; or

$R_1$ is cycloalkyl, alkylcycloalkyl, dialkoxyalkyl, 1,3-dioxolan-2-ylalkyl, 1,3-dioxolan-4-alkyl, 1,3-dioxan-2-ylalkyl, furylalkyl, tetrahydrofurylalkyl or a radical $-NHCOOR_2$, $-CH_2COOR_2$, $-CH(CH_3)COOR_2$ or a radical alkoxyimino-alkyl–$CH(R_3)$–$C(R_4)=NOR_5$, where

$R_2$ is methyl, ethyl, propyl, isopropyl or allyl,

$R_3$ and $R_4$ each individually are hydrogen or $C_1$–$C_4$alkyl and

$R_5$ is hydrogen, $C_1$–$C_4$alkyl, $C_3$–$C_4$alkewnyl or $C_3$–$C_4$alkinyl.

2. An agent according to claim 1, wherein in the acylamide derivative of formula I X represents oxygen.

3. An agent according to claim 1, wherein in the acylamide derivative of formula I R is $C_1$–$C_6$halogenoalkyl or $C_2$–$C_6$halogenoalkylene (sic).

4. An agent according to claim 3, wherein in the acylamide derivative of formula I R is $C_1$–$C_6$halogenoalkyl.

5. An agent according to claim 1, wherein in the acylamide derivative of formula I n is 1, 2 or 3.

6. An agent according to claim 1, wherein in the acylamide derivative of formula I P–X is 4-methoxy.

7. An agent according to claim 1, wherein in the acylamide derivative of formula I P–X is 3,4-dimethoxy.

8. An agent according to claim 1, wherein in the acylamide derivative of formula I P–X is trifluoromethyl.

9. An agent according to claim 1, wherein in the acylamide derivative of formula I P–X is 4-(2,2-dimethoxy)alkyl.

10. An agent according to claim 1, wherein in the acylamide derivative of formula I P–X is 4-(1,3-dioxolan-2-yl)alkyl.

11. An agent according to claim 1, wherein A is $C_1$–$C_8$alkylene.

12. An agent according to claim 1, wherein A is $C_1$–$C_3$alkylene.

13. An agent according to claim 1, wherein $R_1$ is hydrogen.

14. An agent according to claim 1, wherein $R_1$ is $C_1$–$C_6$alkyl or $C_2$–$C_4$alkylene.

15. An agent according to claim 1, wherein $R_1$ is $C_1$–$C_4$alkoxy.

16. An agent according to claim 1, which contains N-(3,4-dimethoxybenzyl)-N-isopropyldichloroacetamide as the acylamide derivative.

17. An agent according to claim 1, which contains N-(3,4-dimethoxyphenylethyl)-N-isopropyldichloroacetamide as the acylamide derivative.

18. An agent according to claim 1, which contains N-(4-trifluoromethylbenzyl)-N-isopropyldichloroacetamide as the acylamide derivative.

19. An agent according to claim 1, which contains N-(3,4-dimethoxybenzyl)-N-isobutyldichloroacetamide as the acylamide derivative.

20. An agent according to claim 1, which contains N-(3,4-dimethoxybenzyl)-N-n-propyldichloroacetamide as the acylamide derivative.

21. An agent according to claim 1, which contains N-(3,4-dimethoxyphenylethyl)-N-n-propyldichloroacetamide as the acylamide derivative.

22. An agent according to claim 1, which contains N-(3,4-dimethoxybenzyl-N-sec-butyldichloroacetamide as the acylamide derivative.

23. An agent according to claim 1, which contains N-(3,4-dimethoxybenzyl)-N-allyl-dichloroacetamide as the acylamide derivative.

24. An agent according to claim 1, which contains N-(4-ethoxy-3-methoxybenzyl)-N-isopropyldichloroacetamide as the acylamide derivative.

25. An agent according to claim 1, which contains N-(3,4-dimethoxybenzyl)-N-(2-methoxyethyl)dichloroacetamide as the acylamide derivative.

26. An agent according to claim 1, which contains N-(3,4-dimethoxybenzyl)-N-isopropyl-(2-chloropropionyl)amide as the acylamide derivative.

27. An agent according to claim 1, which contains N-(4-methoxybenzyl)-N-isopropyldichloroacetamide as the acylamide derivative.

28. An agent according to claim 1, which contains N-(3,4,5-trimethoxybenzyl)-N-isopropyldichloroacetamide as the acylamide derivative.

29. An agent according to claim 1, which contains N-(3,4-dimethoxybenzyl)-N-isopropylchloroacetamide as the acylamide derivative.

30. An agent according to claim 1, which contains N-[2-(4-methoxybenzyl)-2-methyl]ethyl-N-isopropyldichloroacetamide as the acylamide derivative.

31. An agent according to claim 1, which contains $N_1$-(3,4-dimethoxybenzyl)-$N_1$-dichloroacetyl-$N_2$-ethoxycarbonylhydrazide as the acylamide derivative.

32. An agent according to claim 1, which contains N-(3-methoxybenzyl)-N-isopropyldichloroacetamide as the acylamide derivative.

33. An agent according to claim 1, which contains N-(2-methoxybenzyl)-N-isopropyldichloroacetamide as the acylamide derivative.

34. An agent according to claim 1, which contains N-(2,4-dimethoxybenzyl)-N-isopropyldichloroacetamide as the acylamide derivative.

35. An agent according to claim 1, which contains N-(2,5-dimethoxybenzyl)-N-isopropyldichloroacetamide as the acylamide derivative.

36. An agent according to claim 1, which contains N-(2,3-dimethoxybenzyl)-N-isopropyldichloroacetamide as the acylamide derivative.

37. An agent according to claim 1, which contains N-(2,4-dimethoxybenzyl)-N-isopropyldichloroacetamide as the acylamide derivative.

38. An agent according to claim 1, which contains N-[2-(4-methoxybenzyl)-2-methyl]ethylchloroacetamide as the acylamide derivative.

39. An agent according to claim 1, which contains N-(4-methoxybenzyl)-N-(2,2-dimethoxyethyl)dichloroacetamide as the acylamide derivative.

40. An agent according to claim 1, which contains N-(4-methoxybenzyl)-N-ethyldichloroacetamide as the acylamide derivative.

41. An agent according to claim 1, which contains N-4-trifluoromethylbenzylchloroacetamide as the acylamide derivative.

42. An agent according to claim 1, which contains N-(3,4,5-trimethoxyphenylethyl)-N-isopropyldichloroacetamide as the acylamide derivative.

43. An agent according to claim 1, which contains N-[4-(1,3-dioxolan-2-ylmethoxy)benzyl]dichloroacetamide as the acylamide derivative.

44. An agent according to claim 1, which contains N-[4-(1,3-dioxolan-2-ylmethoxy)benzyl]-N-isopropyldichloroacetamide as the acylamide derivative.

45. An agent according to claim 1, which contains N-(3,4-dimethoxybenzyl)-N-methoxyiminoethyldichloroacetamide as the acylamide derivative.

46. An agent according to claim 1, which contains N-(3,4-dimethoxybenzyl)-N-ethoxyiminoethyldichloroacetamide as the acylamide derivative.

47. An agent according to claim 1, which contains N-(3,4-dimethoxybenzyl)-N-methoxyiminoisopropyldichloroacetamide (sic) as the acylamide derivative.

48. An agent according to claim 1, which contains N-(4-trifluoromethylbenzyl)-N-methoxyiminoethyldichloroacetamide as the acylamide derivative.

49. A process for selectively controlling weeds in crops, which comprises treating the crop plants or the area on which they are cultivated with both a herbicidally active chloroacetanilide and an effective amount of an acylamide derivative of the formula I, according to claim 1, as an antidote.

50. The use of the acylamide derivatives of the formula I, according to claim 1, for protecting crop plants against the phytotoxic action of herbicidally active chloroacetanilides.

51. A process for protecting crop plants against damage which occurs on the application of chloroacetanilide herbicides, which comprises treating either the area cultivated for the plant, before or during the application of the herbicide, or the seed or the cuttings of the plants or the plant itself, with an effective amount of an acylamide derivative of the formula I, according to claim 1.

52. The seed of useful plants which has been treated with both a chloroacetanilide herbicide and an amount having an antagonistic action of an acylamide derivative of the formula I, according to claim 1.

## Revendications

1. Agent de protection des plantes cultivées contre l'action phytotoxiques de chloracétanilides à action herbicide, caractérisé en ce qu'il contient outre des supports inertes un herbicide à base de chloracétanilide et comme substance active à action antagoniste un dérivé d'acylamide de formule I

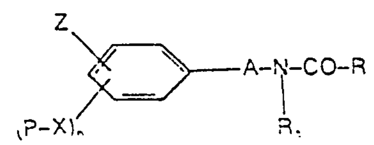

où X représente O ou S, SO, $SO_2$,

P représente alcoyle, alcényle, alcynyle, cycloalcoyle, alcoxyalcoyle, alcénoxyalcoyle, alcynoxyalcoyle, alcoylthioalcoyle, alcénylthioalcoyle, alcynylthioalcoyle, alcoylsulfinylalcoyle, alcoylsulfonylalcoyle, halogénalcoyle, cyanalcoyle, 2,2-dialcoxyalcoyle, 1,3-dioxolan-2-ylalcoyle, 1,3-dioxolan-4-ylalcoyle, 2,2-dialcoyl-1,3-dioxolan-4-ylalcoyle, 1,3-dioxan-yl-2-alcoyle, 2-benzo-pyranyl-alcoyle, alcoxycarbonyle ou alcényloxycarbonyle ou tétrahydrofurfurylalcoyle, le groupe

P–X également un halogénalcoyle

n vaut de 1 à 3

Z représente un hydrogène, alcoyle, un halogène, dioxyméthylène, alcoxy, alcényloxy, alcynyloxy ou cyanoalcoyle,

A représente un radical hydrocarboné en $C_1$ à $C_8$ à chaîne droite, ramifiée ou cyclique, qui peut

être non substitué ou substitué par un alcoyle, alcoylthio, fluor, cyano ou halogénalcoyle,

R représente un halogénalcoyle ou un halogénalcényle,

$R_1$ représente un hydrogène, un radical hydrocarboné en $C_1$ à $C_5$ à chaîne droite ou ramifiée, saturé ou non saturé qui peut être non substitué ou substitué par un alcoxy, polyalcoxy, halogène, cyano ou trifluorométhyle, ou un cycloalcoyle, alcoylcycloalcoyle, dialcoxyalcoyle, 1,3-dioxolan-2-ylalcoyle, 1,3-dioxolan-4-alcoyle, 1,3-dioxan-2-ylalcoyle, furylalcoyle, tétrahydrofurylalcoyle ou un radical $-NHCOOR_2$, $-CH_2COOR_2$, $-CH(CH_3)COOR_2$ ou un radical alcoxy-imino-alcoyle$-CH(R_3)-C(R_4)=NOR_5$, où

$R_2$ représente un méthyle, éthyle, propyle, isopropyle ou allyle,

$R_3$ et $R_4$ représentent chacun un hydrogène ou un alcoyle en $C_1$ à $C_4$ et

$R_5$ représente un hydrogène, alcoyle en $C_1$ à $C_4$, alcényle en $C_3$ à $C_4$ ou alcynyle en $C_3$ à $C_4$.

2. Agent selon la revendication 1, caractérisé en ce que dans le dérivé d'acylamide de formule I, X représente un oxygène.

3. Agent selon la revendication 1, caractérisé en ce que dans le dérivé d'acylamide de formule I, R représente un halogénalcoyle en $C_1$ à $C_6$ ou un halogénalcoylène en $C_2$ à $C_6$.

4. Agent selon la revendication 3, caractérisé en ce que dans le dérivé acylamide de formule I, R représente un halogénalcoyle en $C_1$ à $C_6$.

5. Agent selon la revendication 1, caractérisé en ce que dans le dérivé acylamide de formule I, n vaut 1, 2 ou 3.

6. Agent selon la revendication 1, caractérisé en ce que dans le dérivé acylamide de formule I, P–X représente un 4-méthoxy.

7. Agent selon la revendication 1, caractérisé en ce que dans le dérivé acylamide de formule I, P–X représente un 3,4-diméthoxy.

8. Agent selon la revendication 1, caractérisé en ce que dans le dérivé acylamide de formule I, P–X représente un trifluorométhyle.

9. Agent selon la revendication 1, caractérisé en ce que dans le dérivé acylamide de formule I, P–X représente un 4-(2,2-diméthoxy)alcoyle.

10. Agent selon la revendication 1, caractérisé en ce que dans le dérivé acylamide de formule I, P–X représente un 4-(1,3-dioxolan-2-yl)alcoyle.

11. Agent selon la revendication 1, caractérisé en ce que A représente un alcoylène en $C_1$ à $C_8$.

12. Agent selon la revendication 1, caractérisé en ce que A représente un alcoylène en $C_1$ à $C_3$.

13. Agent selon la revendication 1, caractérisé en ce que $R_1$ représente un hydrogène.

14. Agent selon la revendication 1, caractérisé en ce que $R_1$ représente un alcoyle en $C_1$ à $C_6$ ou un alcoylène en $C_2$ à $C_4$.

15. Agent selon la revendication 1, caractérisé en ce que $R_1$ représente un alcoxy en $C_1$ à $C_4$.

16. Agent selon la revendication 1, caractérisé en ce qu'il contient comme dérivé d'acylamide le N-(3,4-diméthoxybenzyl)-N-isopropyl-dichloroacétamide.

17. Agent selon la revendication 1, caractérisé en ce qu'il contient comme dérivé d'acylamide le N-(3,4-diméthoxyphényléthyl)-N-isopropyl-dichloroacétamide.

18. Agent selon la revendication 1, caractérisé en ce qu'il contient comme dérivé d'acylamide le N-(4-trifluorométhylbenzyl)-N-isopropyl-dichloroacétamide.

19. Agent selon la revendication 1, caractérisé en ce qu'il contient comme dérivé d'acylamide le N-(3,4-diméthoxybenzyl)-N-isobutyldichloroacétamide.

20. Agent selon la revendication 1, caractérisé en ce qu'il contient comme dérivé d'acylamide le N-(3,4-diméthoxybenzyl)-N-n-propyl-dichloroacétamide.

21. Agent selon la revendication 1, caractérisé en ce qu'il contient comme dérivé d'acylamide le N-(3,4-diméthoxyphényléthyl)-N-n-propyl-dichloroacétamide.

22. Agent selon la revendication 1, caractérisé en ce qu'il contient comme dérivé d'acylamide le N-(3,4-diméthoxybenzyl)-N-sec.-butyldichloroacétamide.

23. Agent selon la revendication 1, caractérisé en ce qu'il contient comme dérivé d'acylamide le N-(3,4-diméthoxybenzyl)-N-allyl-dichloroacétamide.

24. Agent selon la revendication 1, caractérisé en ce qu'il contient comme dérivé d'acylamide le N-(4-éthoxy-3-méthoxybenzyl)-N-isopropyl-dichloroacétamide.

25. Agent selon la revendication 1, caractérisé en ce qu'il contient comme dérivé d'acylamide le N-(3,4-diméthoxybenzyl)-N-(2-méthoxyéthyl)-dichloroacétamide.

26. Agent selon la revendication 1, caractérisé en ce qu'il contient comme dérivé d'acylamide le N-(3,4-diméthoxybenzyl)-N-isopropyl-(2-chloropropionyl)-amide.

27. Agent selon la revendication 1, caractérisé en ce qu'il contient comme dérivé d'acylamide le N-(4-méthoxybenzyl)-N-isopropyl-dichloroacétamide.

28. Agent selon la revendication 1, caractérisé en ce qu'il contient comme dérivé d'acylamide le N-(3,4,5-triméthoxybenzyl)-N-isopropyl-dichloracétamide.

29. Agent selon la revendication 1, caractérisé en ce qu'il contient comme dérivé d'acylamide le N-(3,4-diméthoxybenzyl)-N-isopropyl-chloroacétamide.

30. Agent selon la revendication 1, caractérisé en ce qu'il contient comme dérivé d'acylamide le N-(2-(4-méthoxybenzyl)-2-méthyl)éthyl-N-isopropyl-dichloroacétamide.

31. Agent selon la revendication 1, caractérisé en ce qu'il contient comme dérivé d'acylamide le $N_1$-(3,4-diméthoxybenzyl)-$N_1$-dichloroacétyl-$N_2$-éthoxy carbonyl-hydrazide.

32. Agent selon la revendication 1, caractérisé en ce qu'il contient comme dérivé d'acylamide le N-(3-méthoxybenzyl)-N-isopropyl-dichloroacétamide.

33. Agent selon la revendication 1, caractérisé en ce qu'il contient comme dérivé d'acylamide le N-(2-méthoxybenzyl)-N-isopropyl-dichloroacétamide.

34. Agent selon la revendication 1, caractérisé en ce qu'il contient comme dérivé d'acylamide le N-(2,4-diméthoxybenzyl)-N-isopropyl-dichloroacétamide.

35. Agent selon la revendication 1, caractérisé en ce qu'il contient comme dérivé d'acylamide le N-(2,5-diméthoxybenzyl)-N-isopropyl-dichloroacétamide.

36. Agent selon la revendication 1, caractérisé en ce qu'il contient comme dérivé d'acylamide le N-(2,3-diméthoxybenzyl)-N-isopropyl-dichloroacétamide.

37. Agent selon la revendication 1, caractérisé en ce qu'il contient comme dérivé d'acylamide le N-(2,4-diméthoxybenzyl)-N-isopropyl-dichloroacétamide.

38. Agent selon la revendication 1, caractérisé en ce qu'il contient comme dérivé d'acylamide le N-(2-(4-méthoxybenzyl)-2-méthyl)éthyl-chloroacétamide.

39. Agent selon la revendication 1, caractérisé en ce qu'il contient comme dérivé d'acylamide le N-(4-méthoxybenzyl)-N-(2,2-diméthoxyéthyl)-dichloroacétamide.

40. Agent selon la revendication 1, caractérisé en ce qu'il contient comme dérivé d'acylamide le N-(4-méthoxybenzyl)-N-éthyl-dichloroacétamide.

41. Agent selon la revendication 1, caractérisé en ce qu'il contient comme dérivé d'acylamide le N-(4-trifluorométhylbenzyl-chloroacétamide.

42. Agent selon la revendication 1, caractérisé en ce qu'il contient comme dérivé d'acylamide le N-(3,4,5-triméthoxyphényléthyl)-N-isopropyl-dichloroacétamide.

43. Agent selon la revendication 1, caractérisé en ce qu'il contient comme dérivé d'acylamide le N-(4-(1,3-dioxolan-2-yl-méthoxy)benzyl)-dichloroacétamide.

44. Agent selon la revendication 1, caractérisé en ce qu'il contient comme dérivé d'acylamide le N-(4-(1,3-dioxolan-2-yl-méthoxy)benzyl)-N-isopropyl-dichloroacétamide.

45. Agent selon la revendication 1, caractérisé en ce qu'il contient comme dérivé d'acylamide le N-(3,4-diméthoxybenzyl)-N-méthoxyiminoéthyl-dichloroacétamide.

46. Agent selon la revendication 1, caractérisé en ce qu'il contient comme dérivé d'acylamide le N-(3,4-diméthoxybenzyl)-N-éthoxyiminoéthyl-dichloroacétamide.

47. Agent selon la revendication 1, caractérisé en ce qu'il contient comme dérivé d'acylamide le N-(3,4-diméthoxybenzyl)-N-méthoxyiminoisopropyl-dichloroacétamide.

48. Agent selon la revendication 1, caractérisé en ce qu'il contient comme dérivé d'acylamide le N-(4-trifluorométhylbenzyl)-N-méthoxyiminoéthyl-dichloroacétamide.

49. Procédé de lutte sélective contre les mauvaises herbes dans les cultures de plantes utiles, caractérisé en ce qu'on traite les plantes cultivées ou la surface sur laquelle elles sont cultivées aussi bien avec un chloracétanilide à action herbicide qu'avec une quantité efficace d'un dérivé d'acylamide de formule I selon la revendication 1 comme antidote.

50. Application des dérivés d'acylamide de formule I selon la revendication 1 à la protection des plantes cultivées contre l'action nocive des chloracétanilides à action herbicide.

51. Procédé de protection des plantes cultivées contre les atteintes qui apparaissent lors de l'application d'herbicides à base de chloracétanilide, caractérisé en ce qu'on traite les surfaces cultivées des plantes avant ou après l'application de l'herbicide ou les semences ou les boutures des plantes ou les plantes elles-mêmes avec une quantité efficace d'un dérivé d'acylamide de formule I selon la revendication 1.

52. Semences de plantes utiles qui ont été traitées tant avec un herbicide à base de chloracétanilide qu'avec une quantité à action antagoniste d'un dérivé d'acylamide de formule I selon la revendication 1.